# FASCICULE DE BREVET EUROPEEN

(11) **EP 3 941 206 B1**
(45) Date de publication et mention de la délivrance du brevet: **30.09.2026**
(21) Numéro de dépôt: 20710948.9
(22) Date de dépôt: 16.03.2020
(51) Int. Cl.: A01N 63/50, A01N 43/16, A01N 47/04, A01N 59/00, A01P 1/00, A23L 33/00, A61K 31/00, A61L 2/00

(54) **NOUVELLES UTILISATIONS D'UNE LACTONASE MUTEE ET COMPOSITIONS**
NEUE VERWENDUNGEN EINER MUTIERTEN LACTONASE UND ZUSAMMENSETZUNGEN
NEW USES OF A MUTATED LACTONASE, AND COMPOSITIONS

(30) Priorité: 19.03.2019 FR 1902834
(43) Date de publication de la demande: 26.01.2022
(73) Titulaire: Fondation Méditerranée Infection, 13005 Marseille (FR); Gene And Green TK, 13005 Marseille (FR); Aix-Marseille Université, 13284 Marseille Cedex 07 (FR)
(72) Inventeur: CHABRIERE, Eric, 13400 Aubagne (FR); MION, Sonia, 13100 Aix-en-Provence (FR); REMY, Benjamin, 13005 Marseille (FR); PLENER, Laure Claude, 13010 Marseille (FR); DAUDE, David, 13005 Marseille (FR); ELIAS, Mikael Hocine, Minneapolis, 55414 (US)
(74) Mandataire: Grosset-Fournier, Chantal Catherine
(86) Numéro de dépôt international: PCT/EP2020/057159
(87) Numéro de publication internationale: WO 2020/187861

(56) Documents cités:
- WO-A1-2014/167140
- WO-A2-2008/145865

## Description

La présente demande décrit de nouvelles utilisations d'une lactonase mutée et des compositions la contenant.

Certaines bactéries utilisent un système de communication moléculaire appelé Quorum Sensing (QS) afin de coordonner de nombreuses fonctions biologiques telles que la virulence ou la formation de biofilm. Elles utilisent notamment des acyles-homosérines lactones (AHL) comme molécules de communication.

La formation de biofilm bactérien entraîne des problèmes médicaux comme environnementaux. Il est donc important de trouver des solutions pour éliminer efficacement les biofilms bactériens.

On connait les documents suivants : WO2014/167140 qui décrit notamment des phosphotriesterases hyperthermophiles mutées, dont la phosphotriesterase mutée W263I, ayant une activité lactonase améliorée par rapport à une phosphotriesterase hyperthermophile non mutée ainsi que des compositions comprenant une phosphotriesterase mutée et éventuellement un antibiotique et WO2008/145865 qui décrit des phosphotriesterases hyperthermophiles ayant une combinaison de mutations et leurs utilisations. Toutefois, ces documents ne mentionnent pas l'association de ces phosphotriesterases hyperthermophiles mutées avec des agents antimicrobiens pour augmenter la sensibilité des bactéries auxdits agents antimicrobiens.

Ainsi, la présente invention concerne l'utilisation d'une lactonase mutée appartenant à la famille des phosphotriesterase-like lactonase pour augmenter la sensibilité de bactéries aux agents anti-microbiens par rapport à l'utilisation d'agents anti-microbiens seuls,
dans laquelle au moins l'acide aminé tryptophane situé au début de la boucle 8 est substitué par l'acide aminé isoleucine,
et, ladite lactonase mutée ayant la séquence SEQ ID NO : 1 dans laquelle au moins l'acide aminé tryptophane W en position 263 est substitué par l'acide aminé isoleucine I,
et, ladite sensibilité des bactéries aux agents anti-microbiens est augmentée au moins d'un facteur 2,
ledit agent anti-microbien est utilisé à une concentration de 10 µM à 100 mM,
ladite lactonase mutée est utilisée à une concentration de 0,1 mg/L à 10 g/L ou à une concentration de 1/µg/cm² à 1 mg/cm²,
dans laquelle l'agent antimicrobien est choisi parmi le groupe constitué par les antibiotiques ou un mélange d'antibiotiques, les agents désinfectants ou un mélange d'agents désinfectants, les biocides ou un mélange de biocides et les bactériophages éventuellement naturellement présents dans l'environnement ou non, ou un cocktail de tels bactériophages, et
dans laquelle l'utilisation est choisie parmi
   - la prévention et/ou le traitement des infections de plantes,
   - une application sur du matériel contaminé par lesdites bactéries ou susceptible de l'être, et
   - une utilisation comme complément alimentaire pour l'homme ou l'animal ou comme produit de nutrition animale.

Ladite lactonase mutée dérive de la lactonase hyperthermophile de Sulfolobus solfataricus (SsoPox) ou de Saccharolobus solfataricus. La lactonase hyperthermophile de Sulfolobus solfataricus (SsoPox) ou de Saccharolobus solfataricus présente à la fois une activité phosphotriesterase et une activité lactonase. Elle appartient à la famille des phosphotriesterase-like lactonase.

Les enzymes de la famille des phosphotriesterase-like lactonase ont une structure tridimensionnelle conservée. Ainsi, selon la présente invention, ladite lactonase mutée comprend au moins la substitution de l'acide aminé tryptophane situé au début de la boucle 8 des enzymes de la famille des Phosphotriesterase-Like Lactonase par l'acide aminé Isoleucine.

Selon la présente demande, la lactonase mutée peut comprendre d'autres mutations, en plus de la mutation du tryptophane situé au début de la boucle 8 des enzymes de la famille des Phosphotriesterase-Like Lactonase. Ces mutations supplémentaires peuvent, par exemple, améliorer les propriétés de la lactonase mutée ou améliorer sa stabilité.

La présente demande décrit notamment l'utilisation d'une lactonase mutée appartenant à la famille des phosphotriesterase-like lactonase pour augmenter la sensibilité de bactéries aux agents anti-microbiens par rapport à l'utilisation d'agents anti-microbiens seuls,
dans laquelle au moins l'acide aminé tryptophane situé au début de la boucle 8 est substitué par l'acide aminé isoleucine.

Selon un mode de réalisation particulièrement préféré, la lactonase mutée de l'invention dérive de la lactonase hyperthermophile de Sulfolobus solfataricus (SsoPox) ayant la séquence SEQ ID NO : 1 dans laquelle l'acide aminé tryptophane en position 263 est substitué par une Isoleucine I. Ainsi, dans ce mode de réalisation, la lactonase mutée a pour séquence la séquence SEQ ID NO : 2 et comporte une mutation à la position 263 par rapport à la séquence SEQ ID NO : 1.

| | |
|---|---|
| SsoPox Wild-type | MRIPLVGKDSIESKDIGFTLIHEHLRVFSEAVRQQWPHLYNE DEEFRNAVNEVKRAMQFGVKTIVDPTVMGLGRDIRFMEK VVKATGINLVAGTGIYIYIDLPFYFLNRSIDEIADLFIHDIKE GIQGTLNKAGFVKIAADEPGITKDVEKVIRAAAIANKETKV PIITHSNAHNNTGLEQQRILTEEGVDPGKILIGHLGDTDNID YIKKIADKGSFIGLDRYGLDLFLPVDKRNETTLRLIKDGYSD KIMISHDYCCTIDWGTAKPEYKPKLAPRWSITLIFEDTIPFLK RNGVNEEVIATIFKENPKKFFS |
| SEQ ID NO : 1 | |
| SsoPox mutée W263I | MRIPLVGKDSIESKDIGFTLIHEHLRVFSEAVRQQWPHLYNE DEEFRNAVNEVKRAMQFGVKTIVDPTVMGLGRDIRFMEK VVKATGINLVAGTGIYIYIDLPFYFLNRSIDEIADLFIHDIKE GIQGTLNKAGFVKIAADEPGITKDVEKVIRAAAIANKETKV PIITHSNAHNNTGLEQQRILTEEGVDPGKILIGHLGDTDNID YIKKIADKGSFIGLDRYGLDLFLPVDKRNETTLRLIKDGYSD KIMISHDYCCTIDIGTAKPEYKPKLAPRWSITLIFEDTIPFLK RNGVNEEVIATIFKENPKKFFS |
| SEQ ID NO : 2 | |

Le tryptophane situé au début de la boucle 8 correspond au résidu en position 263 de la structure primaire. Toutefois, dans le cas d'autres enzymes de la famille des phosphotriesterase-like lactonase, il peut s'agir d'une position différente dans la structure primaire, mais il s'agira toujours du résidu tryptophane situé au début de la boucle 8 des enzymes de la famille des Phosphotriesterase-Like Lactonase.

Dans un mode de réalisation, la lactonase mutée comprend au moins la substitution du tryptophane par l'isoleucine à la position 263 de sa séquence, comme indiqué dans la séquence SEQ ID NO : 2. En effet, la lactonase mutée peut comprendre d'autres mutations, en plus de la mutation à la position 263 de sa séquence. Ces mutations supplémentaires peuvent, par exemple, améliorer les propriétés de la lactonase mutée de séquence SEQ ID NO : 2 ou améliorer sa stabilité.

Ainsi, lorsqu'un agent anti-microbien donné est utilisé en association avec la lactonase mutée telle que définie précédemment et notamment avec la lactonase mutée de séquence SEQ ID NO : 2, la sensibilité des bactéries à cet agent anti-microbien est augmentée par rapport à l'utilisation de ce même agent anti-microbien seul. Cela signifie que la quantité d'agent anti-microbien nécessaire à l'élimination de ces bactéries ou à l'inhibition de leur croissance peut être diminuée lorsque l'agent anti-microbien est utilisé avec la lactonase mutée telle que définie précédemment et notamment avec la lactonase mutée de séquence SEQ ID NO : 2. Ainsi, la toxicité liée à l'utilisation de concentrations potentiellement élevées d'agent anti-microbien est diminuée. Cela permet également d'éviter le développement de mécanismes de résistance aux agents anti-microbiens par les bactéries.

Afin de mesurer la sensibilité des bactéries aux agents anti-microbiens, il est possible de mesurer, selon les méthodes connues de l'homme du métier :
- la Concentration Minimale d'Inhibition (CMI ou MIC en anglais)
- la Concentration Minimale d'Eradication du Biofilm (CMEB ou MBEC en anglais)
- l'abondance relative des protéines impliquées dans la résistance aux agents anti-microbiens (pompes à efflux, porines)
- l'expression des gènes du système de CRISPR-Cas impliqués dans la résistance aux bactériophages.

Ainsi, pour une dose donnée d'agent anti-microbien, l'observation d'une diminution de la concentration minimale d'inhibition, d'une diminution de la concentration minimale d'éradication du biofilm, d'une variation de l'abondance relative des protéines impliquées dans la résistance aux agents anti-microbiens ou encore d'une altération de l'expression des gènes du système de CRISPR-Cas impliqués dans la résistance aux bactériophages signifie que la sensibilité des bactéries aux agents anti-microbiens est augmentée.

Dans un mode de réalisation, la sensibilité des bactéries aux agents anti-microbiens est augmentée au moins d'un facteur 2 par rapport à l'utilisation desdits agents anti-microbiens seuls.

Cela signifie que, la sensibilité des bactéries aux agents anti-microbiens est augmentée d'un facteur 2, 3, 4, 5, 10, 20, 30, 40, 50, 60, 70, 80, 90 ou 100 par rapport à l'utilisation desdits agents anti-microbiens seuls.

Dans un mode de réalisation particulièrement préféré, la sensibilité des bactéries aux agents anti-microbiens est augmentée d'un facteur allant de 2 à 20 par rapport à l'utilisation desdits agents anti-microbiens seuls.

Le terme « *agent antimicrobien* » désigne un composé qui tue un microorganisme ou inhibe sa croissance.

Dans un mode de réalisation particulier, un agent antimicrobien peut, par exemple, être agent antibactérien, un agent antifongique, un agent antiviral, un agent antiprotozoaire, un agent antiparasitaire ou une combinaison de ces derniers.

Un agent antimicrobien peut, par exemple, être un composé inorganique, un composé organique, une protéine, un anticorps, un sucre, un acide nucléique ou une combinaison de ces derniers.

Dans un mode de réalisation particulier, l'agent anti-microbien peut être choisi parmi le groupe constitué par les antibiotiques ou un mélange d'antibiotiques, les agents désinfectants ou un mélange d'agents désinfectants, les biocides ou un mélange de biocides et les bactériophages éventuellement naturellement présents dans l'environnement ou non, ou un cocktail de tels bactériophages.

Ainsi, dans un mode de réalisation particulier, l'agent antimicrobien est choisi parmi le groupe constitué par les antibiotiques ou un mélange d'antibiotiques, les agents désinfectants ou un mélange d'agents désinfectants, les biocides ou un mélange de biocides et les bactériophages éventuellement naturellement présents dans l'environnement ou non, ou un cocktail de tels bactériophages.

On entend par « *antibiotique* », tout agent capable de tuer une bactérie ou de réduire, limiter ou inhiber sa croissance.

Les antibiotiques peuvent être des antibiotiques bactéricides ou des antibiotiques bactériostatiques. On entend par « *antibiotiques bactéricides* »*,* tout agent capable de tuer une bactérie. On entend par « *antibiotiques bactériostatiques* »*,* tout agent capable de réduire, limiter ou inhiber la croissance bactérienne, sans tuer les bactéries.

On entend par « *désinfectant* », toute substance appliquée sur un objet non-vivant (inerte) ou vivant (comme la peau par exemple) et capable de tuer ou d'inhiber la croissance de microorganismes présents sur l'objet. Un désinfectant à usage corporel, c'est dire appliqué sur les surfaces externes du corps, telle que la peau par exemple, est appelé « *antiseptique* »*.*

On entend par « biocide », toute substance ou préparation destinée à détruire, repousser ou rendre inoffensifs les organismes nuisibles, à prévenir l'action des organismes nuisibles ou à les combattre, par une action chimique ou biologique. En d'autres termes, les biocides sont des substances exerçant une action sur ou contre les organismes nuisibles.

On entend par « *bactériophage* », tout virus capable d'infecter des bactéries. Deux types de bactériophages peuvent être distingués :
- les phages lytiques qui infectent la bactérie, détourne sa machinerie cellulaire pour se reproduire et détruisent la cellule pour libérer de nouveaux phages
- les phages lysogènes, ou tempérés, qui insèrent leur ADN dans celui de la bactérie sous la forme d'un prophage.

On entend par « *bactériophages éventuellement naturellement présents dans l'environnement ou non* »*,* les bactériophages naturellement présents dans l'environnement ainsi que les bactériophages non présents dans l'environnement et ajoutés par un tiers afin d'éliminer des bactéries.

Ledit antibiotique peut être choisi parmi le groupe consistant en : Amikacine, Amoxicilline, Amoxicilline/clavulanate, Ampicilline, Amprolium, Apramycine, Aspoxicilline, Aureomycine, Avilamycine, Azithromycine, Bacitracine, Bambermycine, Baquiloprime, Benzylpenicilline, Bicozamycine, Carbadox, Cefacetrile, Cefalexine, Cefalonium, Cefalotine, Cefapyrine, Cefazoline, Cefdinir, Cefquinome, Ceftiofur, Ceftriaxone, Cefuroxime, Chloramphenicol, Chlortetracycline, Ciprofloxacine, Clarithromycine, Clindamycine, Cloxacilline, Colistine, Dalbavancine, Danofloxacine, Decoquinate, Diclazuril, Dicloxacilline, Difloxacine, Doripenem, Doxycycline, Enramycine, Enrofloxacine, Ertapenem, Erythromycine, Florfenicol, Flumequine, Fosfomycine, Framycetine, acide fusidique, Gentamicine, Gentamicine Sulfate, Gramicidine, bromhydrate d'halofuginone, Hetacilline, Imipenem, Imipenem/cilastatine, Josamycine, Kanamycine, Kitasamycine, Laidlomycine, Lasalocide , Levofloxacine, Lincomycine, chlorhydrate de lincomycine, Maduramycine, Marbofloxacine, Mecillinam, Meropeneme, Miloxacine, Minocycline, Mirosamycine, Monensine, Moxifloxacine, Nafcilline, acide nalidixique, Narasine, Neomycine, Neomycine/oxytetracycline, Neosporine, Nicarbazine, Norfloxacine, Novobiocine, Ofloxacine, Orbifloxacine, Oritavancine, Oxacilline, acide oxolinique , Oxytetracycline, Paromomycine, hydroxyde de penethamate, Penicilline, Penicilline G Potassium, Penicilline procaine, Penicilline V potassium, Phenethicilline, Phenoxymethylpenicilline, Pirlimycine, Polymyxine, Polymyxine B, Polysporine (bacitracine/polymyxine), Pristinamycine, Rifampicine, Rifaximine, Roxarsone, Salinomycine, Semduramicine, Spectinomycine, Spiramycine, Streptomycine, Sulfachlorpyridazine, Sulfadiazine, Sulfadimerazine, Sulfadimethoxazole, Sulfadimethoxine, Sulfadimethoxine et ormetoprim 5:3, Sulfadimidine, Sulfadoxine, Sulfafurazole, Sulfaguanidine, Sulfamethazine, Sulfamethoxazole/trimethoprime, Sulfamethoxine, Sulfamethoxypyridazine, Sulfamonomethoxine, Sulfanilamide, Sulfaquinoxaline, Sulfasalazine, Sulfisoxazole, Surfactine, Telavancine, Terdecamycine, Tetracycline, Thiamphenicol, Tiamuline, Ticarcilline, Tilmicosine, Tobicilline, Tobramycine, Trimethoprime, Trimethoprime/Sulfonamide, Tulathromycine, Tylosine, Valnemuline, Vancomycine, Virginiamycine.

Ledit agent désinfectant peut comprendre un alcool, un chlore, un aldéhyde, un agent oxydant, un iode, un ozone, un composé phénolique, un composé d'ammonium quaternaire ou un mélange de deux ou plus de ces derniers.

Ledit agent désinfectant peut comprendre du formaldéhyde, de l'orthophtalaldéhyde, du glutaraldéhyde, du citrate de dihydrogène d'argent, du polyaminopropyle biguanide, du bicarbonate de sodium, de l'acide lactique, un agent de blanchiment chloré, du méthanol, de l'éthanol, du n-propanol, du 1-propanol, du 2-propanol, de l'isopropanol, un hypochlorite, du dioxyde de chlore, du di chloro isocyanurate, du mono chloro isocyanurate, de l'hydantoïne, du sodium hypochlorite, de l'hypochlorite de calcium, du di chloro isocyanurate de sodium, du chlorite de sodium, du 4-méthylbenzènesulfonamide, du sel de sodium, de l'alcool dichloro-2,4 benzylique, de l'acide performique, de l'acide paracétique, du permanganate de potassium, du peroxymonosulfate de potassium, du phénol, du phénylphénol, du chloroxylénol, de l'hexachlorophène, du thymol, de l'amylmétacrésol, du benzalkonuim chlorure, du bromure de cétyltriméthylammonium, du chlorure de cétylpyridinium, du benzéthonium chloride, de l'acide borique, du vert brillant, du gluconate de chlorhexidine, du providone iodée, du mercurochrome, du miel de manuka, du dichlorhydrate d'octénidine, du polyhexaméthylène biguanide, du baume du Pérou, du peroxyde d'hydrogène, du peroxyde organique, du peroxyacide, de l'hydroperoxyde organique, du sel du peroxyde, des peroxydes d'acide et des mélanges de deux ou plusieurs de ces derniers.

Ledit biocide peut être choisi parmi le groupe consistant en : des peroxydes biocides actifs tels que le peroxyde d'hydrogène, les alcools mono et polyfonctionnels, les aldéhydes, les acides, l'ozone, les composés naphta et les composés contenant un métal alcalin, un métal de transition, un métal du groupe III ou du groupe IV, un soufre, un azote ou un atome d'halogène et des mélanges de deux ou plusieurs de ces derniers.

Ledit biocide est choisi dans le groupe consistant en : formaldéhyde, glutaraldéhyde, acide peracétique, hypochlorites de métaux alcalins, composés d'ammonium quaternaire, 2-amino-2-méthyl-1-propanol, bromure de cétyltriméthylammonium, chlorure de cétylpyridinium, 2,4,4-trichloro-2-hydroxy diphényléther, 1-(4-chlorophényl)-3-(3,4-dichlorophényl) urée, oxyde de zinc, ricinoléate de zinc, pentachlorophénol, naphténate de cuivre, oxyde de tributylétain, dichlorophène, p-nitrophénol, p-chloro-m-xylenol, bêta-naphtol, 2,3,5,6-tétrachloro-4-(méthylsulfonyl) pyridine, salicylanilide, acide bromoacétique, acétate d'ammonium quaternaire d'alkyle, thiosalicylate d'éthyl mercure de sodium, orthophénylphénate de sodium, n-alkyl (C2 à Cs) chlorure de diméthyl benzyl ammonium, les organoborates, 2,2-(1-méthyltriméthylène dioxy)-bis-(4-méthyl-1,3,2-dioxaborinane), 2,2-oxybis(4,4,6-triméthyl)-1,3,2-dioxaborinane, éther monométhylique d'éthylèneglycol, parahydroxybenzoates, composés organiques du bore, 8-hydroxyquinoléine, pentachlorophénate de sodium, chlorure d'alkyl diméthyl éthyl benzyl ammonium, sels d'alkylammonium, 1,3,5-triéthylhexahydro-1,3,5-triazine, chromate de strontium, phénols halogénés, 2-bromo-4-phénylphénol, sels d'argent tels que le nitrate d'argent, le chlorure d'argent, l'oxyde d'argent et l'argent élémentaire, peroxydes organiques, sulfadiazine argentique, dichloro-S-triazinetrione de sodium, 4-chloro-2-cyclohexylphénol, 2-chloro-4-nitrophénol, substitut de paraffines, 3-chloro-3-nitro-2-butanol, stéarate de 2-chloro-2-nitro-1-butanol, acétate de 2-chloro-2-nitrobutyle, 4-chloro-4-nitro-3-hexanol, 1-chloro-1-nitro-1-propanol, 2-chloro-2-nitro-1-propanol, chlorure de triéthylétain, 2,4,5-trichlorophénol, 2,4,6-trichlorophénol, 1,3-dichloro-5,5-diméthylhydantoïne, tris(hydoxyméthyl)nitrométhane, nitroparaffines, 2-nitro-2-éthyl-1,3-propanediol, 2-éthyl-2-nitro-1,3-propanediol, 2-méthyl-2-nitro-1,3-propanediol, hexahydro-1,3,5-tris(2-hydroxyéthyl)-S-triazine, hexahydro-1,3,5-tris(tétrahydro-2-furanyl)-méthyl-S-triazine, bis(thiocyanate) de méthylène, 2,2-dibromo-3-nitrilopropionamide, Béta-bromo-3-nitrostyrène, composés fluorés, N-éthyl-N-méthyl-4-(trifluorométhyl)-2-(3,4-diméthoxyphényl) benzamide, pentachlorophénol, dichlorophène, orthophénylphénol, dibicyclo (3,1,1 ou 2,2,1)-heptyle polyamines, di-bicyclo- (3,1,1 ou 2,2,1)-heptanyle polyamines et des mélanges de deux ou plusieurs de ces derniers.

Ledit bactériophage peut appartenir à la famille des Myoviridae, Siphoviridae, Podoviridae, Corticoviridae, Cystoviridae, Inoviridae, Leviviridae, Microviridae, Plasmaviridae et Tectiviridae ou un cocktail de ces derniers.

La nature de l'agent anti-microbiens à utiliser dépend de la nature de la bactérie à éliminer.

Le terme « *bactérie* », désigne un genre de microorganismes procaryotes scientifiquement classés comme tels. La plupart des bactéries peuvent être classées comme bactéries à Gram positif ou à Gram négatif.

Les bactéries peuvent être choisies parmi les bactéries à gram positif et les bactéries à gram négatif.

Les « *bactéries à Gram positif* » sont des bactéries liées par une seule membrane lipidique et contenant une couche épaisse de peptidoglycanes (20 à 80 nm) qui retient la coloration au cristal violet dans une technique de coloration de Gram.

Les « *bactéries à Gram négatif* » sont des bactéries liées par une membrane cytoplasmique ainsi que par une membrane cellulaire externe, ne contenant qu'une fine couche de peptidoglycanes entre les deux membranes, ce qui ne permet pas de retenir le colorant cristal violet dans une technique de coloration de Gram.

Lesdites bactéries peuvent être choisies parmi le groupe consistant en : Acinetobacter baumannii, Aerococcus viridans, Aeromonas caviae, Aeromonas hydrophila, Aeromonas jandaei, Aeromonas salmonicida, Aeromonas sobria, Aeromonas veronii, Agrobacterium tumefaciens, Aliivibrio fischeri, Aliivibrio salmonicida, Brucella abortus, Brucella canis, Brucella melitensis, Brucella suis, Burkholderia cepacia complex, Burkholderia pseudomallei, Burhkolderia mallei, Chlamydia trachomatis, Chromobacterium violaceum, Clavibacter michiganensis, Clostridium botulinum, Clostridium difficile, Comamonas acidovorans, Comamonas testosteronii, Delftia acidovorans, Desulfovibrio desulfuricans, Desulfovibrio gigas, Desulfovibrio vulgaris, Dickeya dadanantii, Dickeya solanii, Edwarsiellosis anguillarum, Edwarsiellosis ictaluri, Edwarsiellosis piscicida, Edwarsiellosis tarda, Enterobacterium catenabacteriul, Enterococcus faecalis, Erwinia amylovora, Escherichia coli, Francisella noatunensis, Francisella tularensis, Gallionella ferruginea, Klebsiella pneumoniae, Lactococcus garvieae, Legionella pneumophila, Mycobacterium fortuitum, Mycobacterium marinum, Nocardia asteroids, Nocardia crassostreae, Nocardia seriolae, Pantoea aglomerans, Pantoea ananatis, Pantoea stewartii, Pectobacterium atrosepticum, Pectobacterium carotovorum, Porphyromonas gingivalis, Proteus mirabilis, Pseudomonas aeruginosa, Pseudomonas anguilliseptica, Pseudomonas fluorescens, Pseudomonas savastanoi, Pseudomonas syringae, Renibacterium salmoninarum, Salmonella enterica, Serratia liquefaciens, Serratia marcescens, Shewanella japonica, Staphylococcus aureus, Staphylococcus epidermidis, Streptococcus agalactiae, Streptococcus iniae, Streptococcus mutans, Streptomyces scabiei, Thiobacillus ferooxidans, Vibrio cholerae, Vibrio harveyi, Vibrio parahaemolyticus, Vibrio vulnificus, Xanthomonas campestris, Xanthomonas citri, Xanthomonas oryzae, Xanthomonas translucens, Xylella fastidiosa, Yersinia pestis, Yersinia ruckeri.

Les bactéries peuvent être résistantes au traitement par un ou plusieurs agents antimicrobiens seuls.

Ainsi, dans le cas où les bactéries sont résistantes au traitement par un ou plusieurs agents anti-microbiens donnés, un ou plusieurs autres agents anti-microbiens sont administrés en association avec la lactonase mutée telle que définie précédemment et notamment avec la lactonase mutée de séquence SEQ ID NO : 2. Dans ce cas, l'utilisation de la lactonase mutée telle que définie précédemment et notamment avec la lactonase mutée de séquence SEQ ID NO : 2 permet de diminuer la dose d'agents anti-microbiens nécessaire pour éliminer ces bactéries ou inhiber leur croissance et donc, d'éviter le développement de mécanismes de résistance à ces autres agents anti-microbiens par les bactéries.

Ladite lactonase mutée de l'invention est utilisée à une dose efficace.

On entend par « dose efficace », une dose suffisante de lactonase mutée pour augmenter la sensibilité des bactéries aux agents anti-microbiens.

La dose efficace de lactonase mutée dépend de la nature des agents anti-microbiens utilisés et de la bactérie à éliminer.

La lactonase mutée telle que définie précédemment et notamment avec la lactonase mutée de séquence SEQ ID NO : 2 peut être utilisée à une concentration de 0,1 mg/L à 10 g/L (concentration liquide) ou de 1 µg/cm² à 1 mg/cm² (concentration surfacique en solide).

La lactonase mutée de l'invention peut être utilisée à une concentration de 10 mg/L à 2 g/L. La lactonase mutée de l'invention peut être utilisée à une concentration de 5 µg/cm² à 500 µg/cm².

Ledit agent anti-microbien est utilisé à une dose efficace.

On entend par « *dose efficace* », une dose suffisante d'agents anti-microbiens pour tuer lesdites bactéries ou inhiber leur croissance.

La dose efficace d'agents anti-microbiens dépend de la bactérie à éliminer.

Ledit agent anti-microbien peut être utilisé à une concentration de 10 µM à 100 mM.

Ledit agent anti-microbien peut être utilisé à une concentration de 1 mM à 100 mM.

La dose efficace d'agents anti-microbiens est diminuée au moins d'un facteur 2 par rapport à la dose efficace desdits agents anti-microbiens seuls.

Cela signifie que, la dose efficace d'agents anti-microbiens est diminuée d'un facteur 2, 3, 4, 5, 10, 20, 30, 40, 50, 60, 70, 80, 90 ou 100 par rapport à la dose efficace desdits agents anti-microbiens seuls.

La dose efficace d'agents anti-microbiens est diminuée d'un facteur allant de 2 à 20 par rapport à la dose efficace desdits agents anti-microbiens seuls.

Dans un autre aspect, la demande décrit l'utilisation d'une lactonase mutée, telle que défini dans le premier aspect, pour inhiber la croissance bactérienne.

Ainsi, dans cet autre aspect, et dans un mode de réalisation particulier la demande décrit l'utilisation d'une lactonase mutée appartenant à la famille des phosphotriesterase-like lactonase dans laquelle au moins l'acide aminé tryptophane situé au début de la boucle 8 est substitué par l'acide aminé isoleucine et d'au moins un agent anti-microbien pour inhiber la croissance de bactéries,
dans laquelle l'inhibition de la croissance de bactéries est augmentée au moins d'un facteur 2 par rapport à l'utilisation dudit agent anti-microbien seul.

Dans cet autre aspect, et dans un autre mode de réalisation particulier, la demande décrit l'utilisation d'une lactonase mutée et d'au moins un agent anti-microbien pour inhiber la croissance de bactéries,
ladite lactonase mutée ayant la séquence SEQ ID NO : 1 dans laquelle l'acide aminé W en position 263 est substitué par l'acide aminé isoleucine I,
dans laquelle l'inhibition de la croissance de bactéries est augmentée au moins d'un facteur 2 par rapport à l'utilisation dudit au moins un agent anti-microbien seul.

On appelle « croissance bactérienne » d'une bactérie donnée, l'augmentation en nombre ou en masse de cette bactérie sur une période donnée. La mesure de la croissance bactérienne peut être mesurée selon les méthodes connues de l'Homme du métier.

On parle « *d'inhibition bactérienne* » en cas d'arrêt ou de réduction de la croissance bactérienne. On parle d'inhibition de la croissance bactérienne lorsque la croissance bactérienne a été diminuée d'au moins 10% par rapport aux conditions de croissance normales et jusqu'à 100%.

Dans un mode de réalisation particulier, ladite lactonase mutée a la séquence SEQ ID NO : 2.

Concernant l'agent anti-microbien, les différents modes de réalisation détaillés dans le premier aspect s'appliquent à ce deuxième aspect.

De la même façon, selon cet autre aspect, les bactéries sont telles que détaillées dans les différents modes de réalisation du premier aspect de l'invention.

Ladite lactonase mutée et ledit au moins un agent anti-microbien sont utilisés à une dose efficace. Notamment, la dose efficace d'agent anti-microbiens peut diminuée au moins d'un facteur 2 par rapport à la dose efficace dudit au moins un agent anti-microbien seul.

Dans un autre aspect, la demande décrit l'utilisation d'une lactonase mutée, telle que défini dans le premier aspect, pour augmenter la sensibilité de bactéries vis-à-vis des bactériophages.

Ainsi, dans cet autre aspect, et dans un mode de réalisation particulier, la demande décrit l'utilisation d'une lactonase mutée appartenant à la famille des phosphotriesterase-like lactonase dans laquelle au moins l'acide aminé tryptophane situé au début de la boucle 8 est substitué par l'acide aminé isoleucine et d'au moins un bactériophage pour augmenter la sensibilité des bactéries vis-à-vis des bactériophages, éventuellement naturellement présents dans l'environnement ou non, par rapport à l'utilisation dudit au moins un bactériophage seul.

Dans un mode de réalisation particulier, cet autre aspect décrit l'utilisation d'une lactonase mutée et d'au moins un bactériophage pour augmenter la sensibilité des bactéries vis-à-vis des bactériophages, éventuellement naturellement présents dans l'environnement ou non, par rapport à l'utilisation dudit au moins un bactériophage seul,
ladite lactonase mutée ayant la séquence SEQ ID NO : 1 dans laquelle l'acide aminé W en position 263 est substitué par l'acide aminé isoleucine I.

Ainsi, dans un mode de réalisation particulier, ladite lactonase mutée a la séquence SEQ ID NO : 2.

Comme pour le premier aspect de l'invention, dans un mode de réalisation, la sensibilité des bactéries aux bactériophages est augmentée au moins d'un facteur 2 par rapport à l'utilisation du bactériophage seul.

Ainsi, lorsqu'un bactériophage donné est utilisé en association avec la lactonase mutée telle que définie précédemment et notamment avec la lactonase mutée de séquence SEQ ID NO : 2, la sensibilité des bactéries à ce bactériophage est augmentée par rapport à l'utilisation de ce même bactériophage seul. Cela signifie que la quantité de bactériophage nécessaire à l'élimination de ces bactéries ou à l'inhibition de leur croissance peut être diminuée lorsque le bactériophage est utilisé avec la lactonase mutée telle que définie précédemment et notamment avec la lactonase mutée de séquence SEQ ID NO : 2. Cela permet également d'éviter le développement de mécanismes de résistance aux bactériophages par les bactéries.

Dans un mode de réalisation de cet autre_aspect, ledit bactériophage peut appartenir à la famille des Myoviridae, Siphoviridae, Podoviridae, Corticoviridae, Cystoviridae, Inoviridae, Leviviridae, Microviridae, Plasmaviridae et Tectiviridae ou un cocktail de ces derniers.

De la même façon, selon cet autre aspect, les bactéries sont telles que détaillées dans les différents modes de réalisation du premier aspect de l'invention.

Dans un mode de réalisation particulier, les bactéries peuvent être résistantes au traitement par un bactériophage seul.

Comme pour le premier aspect, ladite lactonase mutée et ledit bactériophage sont utilisés à une dose efficace. Notamment, la dose efficace de bactériophages utilisée peut être diminuée d'au moins d'un facteur 2 par rapport à la dose efficace de bactériophage seul.

Dans un autre aspect, la demande décrit des compositions comprenant une lactonase mutée telle que définie dans le premier aspect.

Ainsi, dans cet autre aspect, et dans un mode de réalisation particulier, la demande décrit une composition comprenant comme principe actif une lactonase mutée appartenant à la famille des phosphotriesterase-like lactonase dans laquelle au moins l'acide aminé tryptophane situé au début de la boucle 8 est substitué par l'acide aminé isoleucine et au moins un agent anti-microbien, la dose efficace d'agent anti-microbiens dans ladite composition étant présente en quantité au moins 2 fois inférieure à celle dudit au moins un agent anti-microbien seul.

Dans cet autre aspect, et dans un mode de réalisation particulier, la demande décrit également une composition comprenant comme principe actif une lactonase mutée et au moins un agent anti-microbien,
ladite lactonase mutée ayant la séquence SEQ ID NO : 1 dans laquelle l'acide aminé W en position 263 est substitué par l'acide aminé isoleucine I,
la dose efficace d'agent anti-microbiens dans ladite composition étant présente en quantité au moins 2 fois inférieure à celle dudit au moins un agent anti-microbien seul.

Ainsi, dans un mode de réalisation particulier, ladite lactonase mutée a la séquence SEQ ID NO : 2.

Ainsi, dans un mode de réalisation particulier, la demande décrit une composition comprenant comme principe actif une lactonase mutée appartenant à la famille des phosphotriesterase-like lactonase dans laquelle au moins l'acide aminé tryptophane situé au début de la boucle 8 est substitué par l'acide aminé isoleucine, et au moins un agent anti-microbien,
et en particulier ladite lactonase mutée ayant la séquence SEQ ID NO : 1 dans laquelle au moins l'acide aminé W en position 263 est substitué par l'acide aminé isoleucine I,
la dose efficace d'agent anti-microbiens dans ladite composition étant présente en quantité au moins deux fois inférieure à celle dudit au moins un agent anti-microbien seul.

Concernant l'agent anti-microbien, les différents modes de réalisation détaillés dans le premier aspect s'appliquent à cet autre aspect.

De la même façon, selon cet autre aspect, les bactéries sont telles que détaillées dans les différents modes de réalisation du premier aspect de l'invention.

Comme pour le premier aspect, ladite lactonase mutée et ledit agent anti-microbien sont utilisés à une dose efficace.

Dans cet autre aspect et selon un mode de réalisation, ledit agent anti-microbien est utilisé à une concentration de 10 µM à 100 mM.

Dans un mode de réalisation particulièrement préféré, ledit agent anti-microbien est utilisé à une concentration de 1 mM à 100 mM.

Ainsi, la demande décrit également une composition telle que définie précédemment dans laquelle ledit agent anti-microbien est utilisé à une concentration de 10 µM à 100 mM, de préférence de 1 mM à 100 mM.

La lactonase mutée telle que définie précédemment et notamment avec la lactonase mutée de séquence SEQ ID NO : 2 peut être utilisée à une concentration de 0,1 mg/L à 10 g/L (concentration liquide) ou de 1 µg/cm² à 1 mg/cm² (concentration surfacique en solide). Dans un mode de réalisation particulièrement préféré, la lactonase mutée telle que définie précédemment et notamment avec la lactonase mutée de séquence SEQ ID NO : 2 peut être utilisée à une concentration de 10 mg/L à 2 g/L.

Dans un mode de réalisation particulièrement préféré, la lactonase mutée telle que définie précédemment et notamment avec la lactonase mutée de séquence SEQ ID NO : 2 peut être utilisée à une concentration de 5 µg/cm² à 500 µg/cm².

Ainsi, la demande décrit également une composition telle que définie précédemment dans laquelle ladite lactonase mutée est utilisée à une concentration de 0,1 mg/L à 10 g/L, de préférence de 10 mg/L à 2 g/L ou à une concentration de 1µg/cm² à 1 mg/cm², de préférence de 5µg/cm² à 500 µg/cm².

Dans un mode de réalisation particulier, ladite lactonase mutée telle que définie précédemment peut être appliquée sur du matériel contaminé par lesdites bactéries ou susceptible de l'être.

Dans un mode de réalisation particulier, ledit matériel contaminé par lesdites bactéries ou susceptible de l'être peut être choisi parmi :
- des dispositifs médicaux tels que des pansements, des cathéters, des endoscopes, des implants, des nébulisateurs
- du matériel médical
- des surfaces immergées telles que des coques de bateaux, des infrastructures portuaires ou pétrolières pouvant être la cible de biofouling ou de biocorrosion,
- des installations industrielles telles que des tours aéroréfrigérées, des systèmes de climatisation, des bioréacteurs, des tuyauteries, des nébulisateurs, des brumisateurs, des bassins.

Dans un mode de réalisation particulier, ladite lactonase mutée telle que définie précédemment peut être utilisée comme produit phytosanitaire pour la prévention et/ou le traitement des infections de plantes telles que le feu bactérien.

Par « *traitement* », on entend le moyen de soigner une pathologie déclarée, dont les symptômes sont visibles. Par « *prévention* », on entend le moyen d'empêcher ladite pathologie de se déclarer.

Dans un mode de réalisation particulier, ladite lactonase mutée telle que définie précédemment peut être utilisée dans une composition qui peut être formulée avec au moins un excipient approprié pour son utilisation sous forme de solution, d'huile, de suspension, d'émulsion, de nanoparticules, de liposomes, de granulés ou de surface fonctionnalisée.

Dans un autre aspect, la demande décrit une méthode de prévention et/ou de traitement de pathologies liées à des infections bactériennes, comprenant l'administration d'une lactonase mutée telle que définie dans le premier aspect et d'au moins un agent anti-microbien.

Ainsi, dans cet autre aspect et dans un mode de réalisation particulier, la demande décrit une méthode de prévention et/ou de traitement de pathologies liées à des infections bactériennes, comprenant l'administration d'une lactonase mutée appartenant à la famille des phosphotriesterase-like lactonase dans laquelle au moins l'acide aminé tryptophane situé au début de la boucle 8 est substitué par l'acide aminé isoleucine et d'au moins un agent anti-microbien, la dose efficace d'agents anti-microbiens dans ladite composition étant présente en quantité au moins 2 fois inférieure à celle dudit au moins agent anti-microbien seul.

Dans cet autre aspect et dans un mode de réalisation particulier, la demande décrit une méthode de prévention et/ou de traitement de pathologies liées à des infections bactériennes, comprenant l'administration d'une lactonase mutée et d'au moins un agent anti-microbien,
ladite lactonase mutée ayant la séquence SEQ ID NO : 1 dans laquelle l'acide aminé W en position 263 est substitué par l'acide aminé isoleucine I,
la dose efficace d'agents anti-microbiens dans ladite composition étant présente en quantité au moins 2 fois inférieure à celle dudit au moins un agent anti-microbien seul.

Dans un mode de réalisation de cet autre aspect, lesdites infections bactériennes peuvent être des infections bactériennes chez les plantes telles que le feu bactérien.

Dans un mode de réalisation de cet autre aspect, lesdites infections bactériennes peuvent être des infections bactériennes chez les animaux telles que des dysbioses.

Dans un mode de réalisation de cet autre aspect, lesdites infections bactériennes peuvent être des infections bactériennes chez l'homme telles que les pneumonies, les maladies nosocomiales, les plaies, les brûlures, les infections oculaires, le pied diabétique, pour le traitement des dysbioses, ou pour le traitement de la plaque dentaire.

Tous les modes de réalisations des différents aspects décrits ci-dessus sont applicables à cet autre aspect.

Les figures et les exemples suivants illustrent la présente demande.

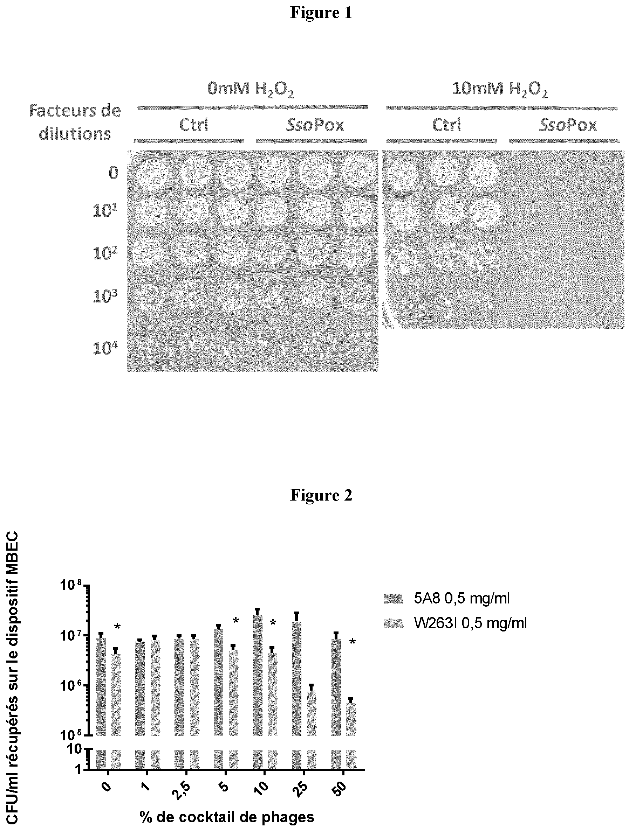

**FIGURE 1** : Dénombrement des bactéries P. aeruginosa récupérées des biofilms formés en présence ou non de la lactonase SsoPox-W263I après traitement à l'antiseptique H2O2.

Gauche : Dénombrement des bactéries P. aeruginosa récupérées des biofilms en absence de traitement à l'antiseptique H2O2, en absence de la lactonase SsoPox-W2631 (Ctrl) ou en présence de la lactonase SsoPox-W263I (SsoPox)

Droite : Dénombrement des bactéries P. aeruginosa récupérées des biofilms après traitement à l'aide de 10mM d'antiseptique H2O2, en absence de la lactonase SsoPox-W263I (Ctrl) ou en présence de la lactonase SsoPox-W263I (SsoPox)

**FIGURE 2** : Augmentation de la sensibilité de Pseudomonas aeruginosa PA14 au cocktail de phages Intesti. Les barres représentent le nombre de bactéries après exposition à différentes concentrations du cocktail de phages (allant de 0 à 50% (vol/vol)), traitées avec la lactonase mutée W263I (0,5 mg/mL) ou avec l'enzyme inactive SsoPox-5A8 (0,5mg/mL).

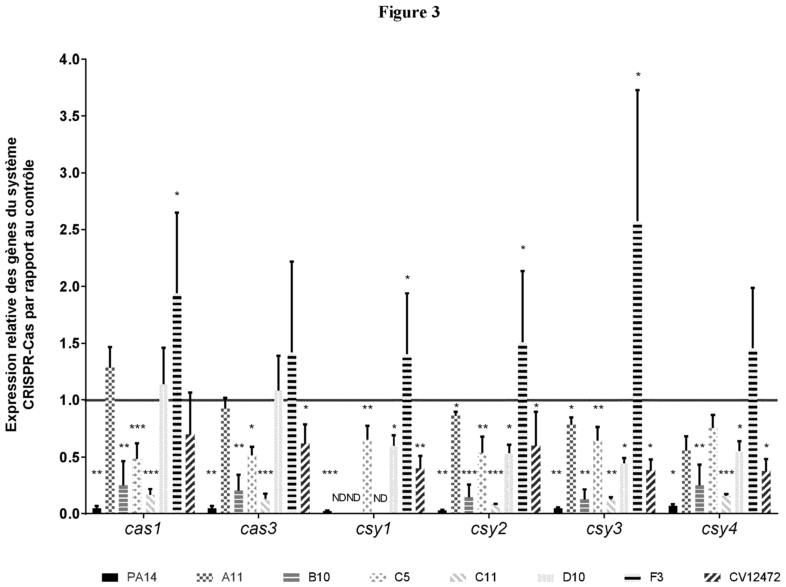

**FIGURE 3** : Modification de l'expression du système CRISPR-Cas. L'expression des gènes cas1, cas3, csy1, csy2, csy3 et csy4 du système CRISPR-Cas a été mesurée pour la souche modèle de P. aeruginosa PA14 ainsi que pour des isolats cliniques issues d'infections du pied diabétique (A11, B10, C5, C11, D10 et F3) et pour la bactérie marine Chromobacterium violaceum CV12472. Les cultures ont été traitées avec l'enzyme SsoPox-W263I ou l'enzyme inactive SsoPox-5A8 (V27G/P67Q/L72C/Y97S/Y99A/T177D/R223L/L226Q/L228M/ W263H). Les histogrammes représentent l'expression des gènes du système CRISPR-Cas traités par SsoPox-W263I normalisés par rapport aux valeurs obtenues avec le variant inactif.

Les barres d'erreurs représentent les variations pour 2 duplicats techniques de 2 réplicats biologiques. *p-values<0.05, **p-values<0.01, ***p-values<0.001 selon le t-test de Student. ND indique que l'expression n'a pas été détectée.

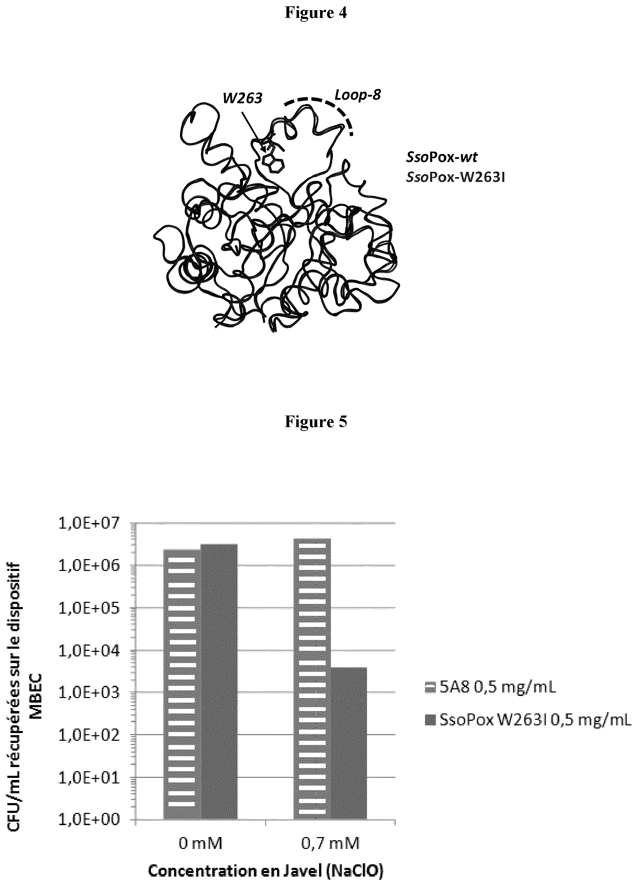

**FIGURE 4** : Structure tridimensionnelle des enzymes de la famille des phosphotriesterase-like lactonase. La structure tridimensionnelle des enzymes de la famille des phosphotriesterase-like lactonase est conservée. Le résidu tryptophane muté correspond au tryptophane situé au début de la boucle 8 des enzymes de la famille des Phosphotriesterase-Like Lactonase. Dans le cas de la présente invention, la lactonase mutée de SEQ ID NO : 2 (SsoPox W263I) résidu tryptophane muté est situé au début de la boucle 8 correspond au tryptophane en position 263 de la structure primaire.

**FIGURE 5** : Dénombrement des bactéries P. aeruginosa récupérées des biofilms formés en présence ou non de la lactonase SsoPox-W263I après traitement à la javel NaOCl.

Gauche : Dénombrement des bactéries P. aeruginosa récupérées des biofilms en absence de traitement à la javel NaOCl, en présence de la lactonase SsoPox-W2631 (barre pleine) ou d'un variant inactif de l'enzyme SsoPox 5A8 (barre hachurée).

Droite : Dénombrement des bactéries P. aeruginosa récupérées des biofilms après traitement à l'aide de 0.7mM de javel NaOCl, en présence de la lactonase SsoPox-W263I (barre pleine) ou d'un variant inactif de l'enzyme SsoPox 5A8 (barre hachurée).

Une diminution de 2 log est observée lorsque la javel (0.7 mM) et la lactonase SsoPox-W263I sont utilisées simultanément démontrant l'effet synergique de la combinaison

### Matériel et Méthode

### a) Tests de sensibilité

Les doses d'agents anti-microbiens nécessaires à l'élimination de biofilms bactériens sont déterminées en utilisant la technique « MBEC (Minimal Biofilm Eradication Concentration) Assay TM » développée par Innovotech (Alberta, Canada) selon les données du fournisseur.

Les biofilms bactériens sont formés par croissance bactérienne dans un milieu et des conditions adaptés aux bactéries étudiées en présence ou non de la lactonase SsoPox-W263I de séquence SEQ ID NO : 2.

Les bactéries sont pré-cultivées pendant 6 heures en flasques oxygénées dans les conditions indiquées dans le tableau 1 puis les plaques de MBEC sont ensemencées en diluant la préculture au 1/1000 en présence ou non de la lactonase SsoPox-W263I à 0,5 mg/mL. Après 24 heures de croissance, les biofilms bactériens formés sur les picots du couvercle de la plaque MBEC sont rincés par immersion pendant 5 minutes dans une solution tampon (tableau 1). Les biofilms sont ensuite immergés dans une solution tampon contenant les agents anti-microbiens (désinfectants, antibiotiques bactéricides ou bactériostatiques, bactériophages, biocides) selon une durée représentative du mécanisme d'action de l'agent anti-microbien étudié (1h30 pour les antiseptiques, 3h pour les antibiotiques, 4h pour les phages). Après immersion dans les agents anti-microbiens, les biofilms bactériens sont rincés pendant 5 minutes dans une solution tampon puis incubés pendant 1 heure dans un milieu nutritif adapté aux bactéries étudiées et contenant des détergents pour détacher les biofilms (tableau 1). Après 1 heure d'incubation les bactéries détachées du biofilm et donc présentes dans les puits de la plaque MBEC sont diluées en série et des étalements sur gélose nutritive adaptée sont réalisés pour effectuer des dénombrements bactériens et déterminer le nombre de bactéries ayant survécu au traitement combiné de l'enzyme mutée SsoPox-W263I et de l'agent anti-microbien (Figure 1). La MBEC est la concentration minimale en agent anti-microbien pour éradiquer les bactéries contenues dans le biofilm.

**Tableau 2 : Conditions expérimentales pour la détermination des MBEC. LB (10 g /L peptone, 5 g/L extrait de levures, 10 g/L NaCl); tampon MOPS 10x (500 mM MOPS, 40 mM Tricine, 500 mM NaCl, 10 mM K2HSO4, 500 mM MgCl2, 100 mM CaCl2, 3 mM (NH4)6Mo7O24, 400 mM H3BO3, 30 mM Co(OAc)2, 10 mM CuSO4, 80 mM MnSO4, 10 mM ZnSO4 [pH 7.0], stérilisé par filtration 0,22 µm); milieu MOPS glutamate (MOPS 1x, 15 mM NH4Cl, 5 µM Fe2SO4, 4 mM K2HPO4, 25 mM glutamate); PBS (8 g/L NaCl, 0,2 g/L KCl, 1,44 g/L Na2HPO4, 0,24 g/L KH2PO4); Recovery LB (LB, 20 g/L saponine, 10 g/L Tween-80).**

| Bactérie | Souche | Conditions préculture | Milieu nutritif MBEC | Température | Agitation | Solution tampon | Milieu de récupération |
|---|---|---|---|---|---|---|---|
| *Pseudomonas aeruginosa* | UCBPP-PA14 | LB - 37°C | MOPS glutamate | 37°C | Orbitale - 110 RPM | MOPS | Recovery LB |
| *Chromobacterium violaceum* | ATCC-12472 | LB - 37°C | LB | 25°C | Orbitale - 110 RPM | PBS | Recovery LB |

### b) Expression des gènes du système CRISPR-Cas

Les bactéries ont été cultivées en milieu MOPS pour P. aeruginosa et LB pour C. violaceum, en présence de la lactonase mutée SsoPox-W263I (0,5 mg/ml) ou de son variant inactif 5A8 (0,5 mg/ml). Après 16 heures de culture (phase stationnaire), les bactéries sont récupérées par centrifugation.

Les ARN ont été extraits et purifiés avec le mini kit RNA PureLink^{®} (ThermoFisher) selon les recommandations du fournisseur puis traités avec la TURBO DNA-free^{™} kit (ThermoFisher) pour éliminer les contaminations par l'ADN génomique. La qualité des échantillons a été vérifiée par migration sur gel d'agarose 1.5% et la quantité d'acide nucléique a été mesurée avec un spectrophotomètre NanoDrop 2000 (Thermo Scientific) à OD260 nm. Les ADN complémentaires (ADNc) ont été synthétisés en utilisant le kit de Reverse transcription reagents TaqMan^{®} (ThermoFisher) selon les recommandations du fabriquant. Des RT-PCR ont ensuite été réalisées en utilisant le kit LuminoCt^{®} SYBR^{®} Green qPCR ReadyMix^{™} et un thermocycleur CFX thermocycler (Bio-Rad) et des paires d'amorces spécifiques. L'amplification par PCR a été réalisée avec la méthode suivante : Dénaturation pendant 5 minutes à 94 °C, suivie de 29 cycles de [1 minute à 94 °C, 1 minute à 55°C, 30 secondes à 72 °C] pour l'amplification, puis une étape d'élongation finale pendant 7 minutes à 72 °C. La fluorescence des échantillons est mesurée à la fin de chaque cycle et les courbes de dénaturation ont été analysées avec le logiciel CFX Manager^{™} software (Bio-Rad). L'expression des gènes a été normalisée par l'expression d'un gène de ménage 5S RNA.

**Tableau 3 : Séquences des amorces utilisées pour évaluer l'expression des gènes du système CRISPR-Cas**

| **Bacterial species** | **Target gene** | **Sequence** | |
|---|---|---|---|
| **P. aeruginosa** | cas1 | Forward | TCAAGGACTCGCTGATCCTG (SEQ ID NO:3) |
| | | Reverse | GATCATGAAGTCCAGGGCCT (SEQ ID NO:4) |
| | cas3 | Forward | GGTTGATCGTCAGCCATCAT (SEQ ID NO:5) |
| | | Reverse | GGCCTTTTCTTTTGCGTCT (SEQ ID NO:6) |
| | csy1 | Forward | TCTTCGAGCATGACTTCGGA (SEQ ID NO:7) |
| | | Reverse | TGGCGAGGTTGTTATGGACT (SEQ ID NO:8) |
| | csy2 | Forward | CGTCCGAAGAAGAAGCATCG (SEQ ID NO:9) |
| | | Reverse | CGCAGCGGTGTTTCTCTATC (SEQ ID NO:10) |
| | csy3 | Forward | AAGACCAAGGACCGTGACC (SEQ ID NO:11) |
| | | Reverse | AGCCCTGATCGTTCACGTAG (SEQ ID NO:12) |
| | csy4 | Forward | ACAGGATCGGCGTGAGCTT (SEQ ID NO:13) |
| | | Reverse | CCGCAACCCTTCCAGCCA (SEQ ID NO:14) |
| | 5S | Forward | GAACCACCTGATCCCTTCCC (SEQ ID NO:15) |
| | | Reverse | TAGGAGCTTGACGATGACCT (SEQ ID NO:16) |
| **C. violaceum** | cas1 | Forward | CAGGATGGCTGCGTCTTTG (SEQ ID NO:17) |
| | | Reverse | AACTACCTGGCCTACGGC (SEQ ID NO:18) |
| | cas3 | Forward | GGACAGGTAGGAGGCTTG (SEQ ID NO:19) |
| | | Reverse | TACGCGAGCAAGTGACCC (SEQ ID NO:20) |
| | csy1 | Forward | GGAATTCCGCCTCCGCCA (SEQ ID NO:21) |
| | | Reverse | GCCGACAGCGATGAAGAC (SEQ ID NO:22) |
| | csy2 | Forward | TCACCGGCCTGATGACGGC (SEQ ID NO:23) |
| | | Reverse | GAAGCGCTGGATGTAGTCG (SEQ ID NO:24) |
| | csy3 | Forward | GCGAGTACAGGCTTTCCAC (SEQ ID NO:25) |
| | | Reverse | AAACCATCTGGCGGCACTC (SEQ ID NO:26) |
| | csy4 | Forward | CGGAAGCATTGGCCGGTG (SEQ ID NO:27) |
| | | Reverse | CGCGCGACAGGCTGATG (SEQ ID NO:28) |
| | 5S | Forward | CTGGTGGCCATAGCGAGG (SEQ ID NO:29) |
| | | Reverse | GTCTGGCGGTGTCCTACTT (SEQ ID NO:30) |

### Résultats

### a) Tests de sensibilité

La Figure 1 montre que sans traitement antiseptique (gauche), autant de bactéries sont récupérées des biofilms qu'il y ait eu ou non un traitement par la lactonase mutée SsoPox-W263I. Après traitement pendant 1 heure 30 avec 10 mM d'antiseptique H2O2, aucune bactérie n'est récupérée du biofilm avec l'utilisation de la lactonase mutée SsoPox-W263I alors que 104 à 105 cellules bactériennes sont récupérées sans lactonase. Dans l'échantillon contrôle (ctrl) réalisé avec un variant inactif de SsoPox-5A8 (V27G / P67Q / L72C / Y97S / Y99A / T177D / R223L / L226Q / L228M / W263H), il faut 100 mM d'antiseptique pour éradiquer entièrement le biofilm.

Cela signifie que l'utilisation d'un antiseptique et d'une lactonase mutée SsoPox-W263I permet de diminuer considérablement le nombre de bactéries récupérées du biofilm par rapport à l'utilisation de l'antiseptique seul ou de la lactonase mutée SsoPox-W263I seule.

Les résultats obtenus dans le tableau 3 montrent que la présence de la lactonase mutée SsoPox-W263I permet de diminuer d'un facteur 10 la concentration nécessaire en antibiotique et antiseptique (gentamicine, tobramycine et H2O2) pour éliminer les biofilms de P. aeruginosa. La même tendance, avec un facteur d'au moins 20 est observé pour un biofilm de la bactérie marine C. violaceum traité par un biocide utilisé dans les peintures antifouling de coque de bateaux.

L'utilisation préventive de la lactonase mutée SsoPox-W263I, en plus du biocide, permet donc de réduire de manière significative l'utilisation de produits biocides qui ont un impact négatif sur l'environnement et qui sont connus pour faciliter l'émergence de bactéries résistantes en milieu hospitalier ou naturel.

**Tableau 4 : MBEC avec ou sans utilisation de la lactonase mutée SsoPox W263I**

| **Bactérie** | **Souche** | **Type de biocide** | **Nom du biocide** | **SsoPox W263I** | **MBEC** |
|---|---|---|---|---|---|
| *Pseudomonas aeruginosa* | UCBPP-PA14 | Antibiotique | Gentamicine | - | **20 µg/mL** |
| | | | | 0.5 mg/mL | **2 µg/mL** |
| *Pseudomonas aeruginosa* | UCBPP-PA14 | Antibiotique | Tobramycine | - | **10 µg/mL** |
| | | | | 0.5 mg/mL | **1 µg/mL** |
| *Pseudomonas aeruginosa* | UCBPP-PA14 | Antiseptique | H2O2 | - | **100 mM** |
| | | | | 0.5 mg/mL | **10 mM** |
| *Chromobacterium violaceum* | ATCC-12472 | Biocide large spectre | Preventol A4S (Dichlofluanide) | - | **<200 µM** |
| | | | | 0.5 mg/mL | **>10 µM** |

Par ailleurs, les bactéries Pseudomonas aeruginosa sont également traitées avec la lactonase mutée W263I et un cocktail de bactériophages (Instesti cocktail ; Microgen Russia) avec des résultats satisfaisants.

La bactérie P. aeruginosa PA14 est traitée par l'enzyme mutée SsoPox-W2631 et le cocktail de phages Intesti ou par le cocktail de phages Intesti et le variant inactif SsoPox-5A8. Le cocktail de phages Intesti consiste en un mélange de filtrats stériles de phages dirigés contre les bactéries Shigella flexneri (sérovariants 1, 2, 3, 4, 6), Shigellasi, Proteus vulgaris, Proteus mirabilis, Enterococcus, Staphylococcus, Pseudomonas aeruginosa et d'excipients tel que le sulfate de 8-hydroxyquinoléine monohydraté à 0,0001 g / ml (teneur estimée) et est commercialisé par Intesti-bacteriophage, Microgen, Russia
La figure 2 montre que la bactérie P. aeruginosa PA14 traitée par l'enzyme mutée SsoPox-W263I et le cocktail de phages Intesti est plus sensible au cocktail de phages Intesticontrairement à la bactérie traitée par le variant inactif SsoPox-5A8 et le cocktail de phages Intesti. En effet, la bactérie traitée par l'enzyme inactive SsoPox-5A8 et le cocktail de phages est peu impactée par le cocktail de phages Intesti alors que moins de bactéries sont dénombrées après traitement par l'enzyme mutée SsoPox-W263I.

### b) Expression des gènes du système CRISPR-Cas

Le système CRISPR-Cas est impliqué dans la défense des bactéries vis-à-vis des bactériophages. Pour déterminer si l'enzyme mutée SsoPox-W263I a un effet sur la régulation du système CRISPR-Cas, les niveaux d'expression des gènes cas1, cas3, csy1, csy2, csy3 et csy4 du système CRISPR-Cas ont été mesurés chez P. aeruginosa PA14 et des isolats cliniques de P. aeruginosa issus d'infections du pied diabétique (A11, B10, C5, C11, D10, F3) ainsi que chez la souche marine de Chromobacterium violaceum CV12472. Des amorces ciblant ces différents gènes ont été créées à partir des génomes de P. aeruginosa PA14 et C. violaceum CV12472 (tableau 2). Les cultures ont été traitées avec l'enzyme SsoPox-W263 ou l'enzyme inactive SsoPox-5A8 (V27G/P67Q/L72C/Y97S/Y99A/T177D/R223L/L226Q/L228M/W263H).

L'expression de gènes est totalement abolie chez P. aeruginosa PA14 après traitement par l'enzyme mutée SsoPox-W263I. Chez B10 et C11 l'expression des gènes est diminuée d'un facteur 5.5 et 8 respectivement. Chez A11 et D10 l'expression des gènes csy1-4 est réduite significativement. Chez F3 à l'inverse l'expression des gènes est augmentée d'un facteur 1.7 en moyenne. Chez C. violaceum l'expression des gènes cas3 et csy2-4 est significativement diminuée. Ces résultats montrent que l'enzyme SsoPox-W263I impacte la régulation du système CRISPR-Cas.

### c) Démonstration d'un effet synergique entre la lactonase mutée W263I et un biocide (NaOCl)

Il est montré à la Figure 5, que sans traitement à l'hypochlorite de sodium (NaOCl) (gauche), autant de bactéries sont récupérées des biofilms qu'il y ait eu ou non un traitement par la lactonase mutée W263I. Après traitement avec 0,7 mM d'hypochlorite de sodium et la lactonase mutée W263I, le nombre de bactéries récupérées des biofilms a été diminué de 2 Log par rapport à l'utilisation de l'hypochlorite de sodium seul.

Ces résultats montrent que l'utilisation de l'hypochlorite de sodium en association avec la lactonase mutée W263I permet de diminuer considérablement le nombre de bactéries récupérées du biofilm par rapport à l'utilisation de l'hypochlorite de sodium seul ou de la lactonase mutée W263I seule, montrant ainsi l'existence d'un effet synergique entre l'hypochlorite de sodium et la lactonase mutée W263I.

## Revendications

1. Utilisation d'une lactonase mutée appartenant à la famille des phosphotriesterase-like lactonase pour augmenter la sensibilité de bactéries aux agents anti-microbiens par rapport à l'utilisation d'agents anti-microbiens seuls,
dans laquelle au moins l'acide aminé tryptophane situé au début de la boucle 8 est substitué par l'acide aminé isoleucine,
et, ladite lactonase mutée ayant la séquence SEQ ID NO : 1 dans laquelle au moins l'acide aminé tryptophane W en position 263 est substitué par l'acide aminé isoleucine I,
et, ladite sensibilité des bactéries aux agents anti-microbiens est augmentée au moins d'un facteur 2,
ledit agent anti-microbien est utilisé à une concentration de 10 µM à 100 mM,
ladite lactonase mutée est utiliséé à une concentration de 0,1 mg/L à 10 g/L ou à une concentration de 1µg/cm² à 1 mg/cm²,
dans laquelle l'agent antimicrobien est choisi parmi le groupe constitué par les antibiotiques ou un mélange d'antibiotiques, les agents désinfectants ou un mélange d'agents désinfectants, les biocides ou un mélange de biocides et les bactériophages éventuellement naturellement présents dans l'environnement ou non, ou un cocktail de tels bactériophages, et
dans laquelle l'utilisation est choisie parmi
- la prévention et/ou le traitement des infections de plantes,
- une application sur du matériel contaminé par lesdites bactéries ou susceptible de l'être, et
- une utilisation comme complément alimentaire pour l'homme ou l'animal ou comme produit de nutrition animale.

2. Utilisation d'une lactonase selon la revendication 1, dans laquelle ledit antibiotique est choisi parmi le groupe consistant en : Amikacine, Amoxicilline, Amoxicilline/clavulanate, Ampicilline, Amprolium, Apramycine, Aspoxicilline, Aureomycine, Avilamycine, Azithromycine, Bacitracine, Bambermycine, Baquiloprime, Benzylpenicilline, Bicozamycine, Carbadox, Cefacetrile, Cefalexine, Cefalonium, Cefalotine, Cefapyrine, Cefazoline, Cefdinir, Cefquinome, Ceftiofur, Ceftriaxone, Cefuroxime, Chloramphenicol, Chlortetracycline, Ciprofloxacine, Clarithromycine, Clindamycine, Cloxacilline, Colistine, Dalbavancine, Danofloxacine, Decoquinate, Diclazuril, Dicloxacilline, Difloxacine, Doripenem, Doxycycline, Enramycine, Enrofloxacine, Ertapenem, Erythromycine, Florfenicol, Flumequine, Fosfomycine, Framycetine, acide fusidique, Gentamicine, Gentamicine Sulfate, Gramicidine, bromhydrate d'halofuginone, Hetacilline, Imipenem, Imipenem/cilastatine, Josamycine, Kanamycine, Kitasamycine, Laidlomycine, Lasalocide , Levofloxacine, Lincomycine, chlorhydrate de lincomycine, Maduramycine, Marbofloxacine, Mecillinam, Meropeneme, Miloxacine, Minocycline, Mirosamycine, Monensine, Moxifloxacine, Nafcilline, acide nalidixique, Narasine, Neomycine, Neomycine/oxytetracycline, Neosporine, Nicarbazine, Norfloxacine, Novobiocine, Ofloxacine, Orbifloxacine, Oritavancine, Oxacilline, acide oxolinique , Oxytetracycline, Paromomycine, hydroxyde de penethamate, Penicilline, Penicilline G Potassium, Penicilline procaine, Penicilline V potassium, Phenethicilline, Phenoxymethylpenicilline, Pirlimycine, Polymyxine, Polymyxine B, Polysporine (bacitracine/polymyxine), Pristinamycine, Rifampicine, Rifaximine, Roxarsone, Salinomycine, Semduramicine, Spectinomycine, Spiramycine, Streptomycine, Sulfachlorpyridazine, Sulfadiazine, Sulfadimerazine, Sulfadimethoxazole, Sulfadimethoxine, Sulfadimethoxine et ormetoprim 5:3, Sulfadimidine, Sulfadoxine, Sulfafurazole, Sulfaguanidine, Sulfamethazine, Sulfamethoxazole/trimethoprime, Sulfamethoxine, Sulfamethoxypyridazine, Sulfamonomethoxine, Sulfanilamide, Sulfaquinoxaline, Sulfasalazine, Sulfisoxazole, Surfactine, Telavancine, Terdecamycine, Tetracycline, Thiamphenicol, Tiamuline, Ticarcilline, Tilmicosine, Tobicilline, Tobramycine, Trimethoprime, Trimethoprime/Sulfonamide, Tulathromycine, Tylosine, Valnemuline, Vancomycine, Virginiamycine.

3. Utilisation d'une lactonase selon la revendication 1, dans laquelle ledit agent désinfectant comprend un alcool, un chlore, un aldéhyde, un agent oxydant, un iode, un ozone, un composé phénolique, un composé d'ammonium quaternaire ou un mélange de deux ou plus de ces derniers,
de préférence, ledit agent désinfectant comprend du formaldéhyde, de l'orthophtalaldéhyde, du glutaraldéhyde, du citrate de dihydrogène d'argent, du polyaminopropyle biguanide, du bicarbonate de sodium, de l'acide lactique, un agent de blanchiment chloré, du méthanol, de l'éthanol, du n-propanol, du 1-propanol, du 2-propanol, de l'isopropanol, un hypochlorite, du dioxyde de chlore, du di chloro isocyanurate, du mono chloro isocyanurate, de l'hydantoïne, du sodium hypochlorite, de l'hypochlorite de calcium, du di chloro isocyanurate de sodium, du chlorite de sodium, du 4-méthylbenzènesulfonamide, du sel de sodium, de l'alcool dichloro-2,4 benzylique, de l'acide performique, de l'acide paracétique, du permanganate de potassium, du peroxymonosulfate de potassium, du phénol, du phénylphénol, du chloroxylénol, de l'hexachlorophène, du thymol, de l'amylmétacrésol, du benzalkonuim chlorure, du bromure de cétyltriméthylammonium, du chlorure de cétylpyridinium, du benzéthonium chloride, de l'acide borique, du vert brillant, du gluconate de chlorhexidine, du providone iodée, du mercurochrome, du miel de manuka, du dichlorhydrate d'octénidine, du polyhexaméthylène biguanide, du baume du Pérou, du peroxyde d'hydrogène, du peroxyde organique, du peroxyacide, de l'hydroperoxyde organique, du sel du peroxyde, des peroxydes d'acide et des mélanges de deux ou plusieurs de ces derniers.

4. Utilisation d'une lactonase selon la revendication 1, dans laquelle le biocide est choisi parmi le groupe consistant en : des peroxydes biocides actifs tels que le peroxyde d'hydrogène, les alcools mono et polyfonctionnels, les aldéhydes, les acides, l'ozone, les composés naphta et les composés contenant un métal alcalin, un métal de transition, un métal du groupe III ou du groupe IV, un soufre, un azote ou un atome d'halogène et des mélanges de deux ou plusieurs de ces derniers,
de préférence, ledit biocide est choisi dans le groupe consistant en : formaldéhyde, glutaraldéhyde, acide peracétique, hypochlorites de métaux alcalins, composés d'ammonium quaternaire, 2-amino-2-méthyl-1-propanol, bromure de cétyltriméthylammonium, chlorure de cétylpyridinium, 2,4,4-trichloro-2-hydroxy diphényléther, 1-(4-chlorophényl)-3-(3,4-dichlorophényl) urée, oxyde de zinc, ricinoléate de zinc, pentachlorophénol, naphténate de cuivre, oxyde de tributylétain, dichlorophène, p-nitrophénol, p-chloro-m-xylenol, bêta-naphtol, 2,3,5,6-tétrachloro-4-(méthylsulfonyl) pyridine, salicylanilide, acide bromoacétique, acétate d'ammonium quaternaire d'alkyle, thiosalicylate d'éthyl mercure de sodium, orthophénylphénate de sodium, n-alkyl (C2 à Cs) chlorure de diméthyl benzyl ammonium, les organoborates, 2,2-(1-méthyltriméthylène dioxy)-bis-(4-méthyl-1,3,2-dioxaborinane), 2,2-oxybis(4,4,6-triméthyl)-1,3,2-dioxaborinane, éther monométhylique d'éthylèneglycol, parahydroxybenzoates, composés organiques du bore, 8-hydroxyquinoléine, pentachlorophénate de sodium, chlorure d'alkyl diméthyl éthyl benzyl ammonium, sels d'alkylammonium, 1,3,5-triéthylhexahydro-1,3,5-triazine, chromate de strontium, phénols halogénés, 2-bromo-4-phénylphénol, sels d'argent tels que le nitrate d'argent, le chlorure d'argent, l'oxyde d'argent et l'argent élémentaire, peroxydes organiques, sulfadiazine argentique, dichloro-S-triazinetrione de sodium, 4-chloro-2-cyclohexylphénol, 2-chloro-4-nitrophénol, substitut de paraffines, 3-chloro-3-nitro-2-butanol, stéarate de 2-chloro-2-nitro-1-butanol, acétate de 2-chloro-2-nitrobutyle, 4-chloro-4-nitro-3-hexanol, 1-chloro-1-nitro-1-propanol, 2-chloro-2-nitro-1-propanol, chlorure de triéthylétain, 2,4,5-trichlorophénol, 2,4,6-trichlorophénol, 1,3-dichloro-5,5-diméthylhydantoïne, tris(hydoxyméthyl)nitrométhane, nitroparaffines, 2-nitro-2-éthyl-1,3-propanediol, 2-éthyl-2-nitro-1,3-propanediol, 2-méthyl-2-nitro-1,3-propanediol, hexahydro-1,3,5-tris(2-hydroxyéthyl)-S-triazine, hexahydro-1,3,5-tris(tétrahydro-2-furanyl)-méthyl-S-triazine, bis(thiocyanate) de méthylène, 2,2-dibromo-3-nitrilopropionamide, Béta-bromo-3-nitrostyrène, composés fluorés, N-éthyl-N-méthyl-4-(trifluorométhyl)-2-(3,4-diméthoxyphényl) benzamide, pentachlorophénol, dichlorophène, orthophénylphénol, di-bicyclo (3,1,1 ou 2,2,1)-heptyle polyamines, dibicyclo- (3,1,1 ou 2,2,1)-heptanyle polyamines et des mélanges de deux ou plusieurs de ces derniers.

5. Utilisation d'une lactonase selon la revendication 1, dans laquelle le bactériophage appartient à la famille des Myoviridae, Siphoviridae, Podoviridae, Corticoviridae, Cystoviridae, Inoviridae, Leviviridae, Microviridae, Plasmaviridae et Tectiviridaeou un cocktail de ces derniers.

6. Utilisation d'une lactonase selon la revendication 1, dans laquelle la dose efficace d'agent anti-microbiens est diminuée au moins d'un facteur 2 par rapport à la dose efficace d'agent anti-microbien seul.

7. Utilisation d'une lactonase selon la revendication 1, dans laquelle la dose efficace d'agent anti-microbiens dans ladite composition étant présente en quantité au moins deux fois inférieure à celle dudit au moins un agent anti-microbien seul.

8. Utilisation d'une lactonase selon la revendication 1, dans laquelle ledit agent anti-microbien est utilisé à une concentration de 1 mM à 100 mM.

9. Utilisation d'une lactonase selon la revendication 1, dans laquelle ladite lactonase mutée est utilisée à une concentration de 10 mg/L à 2 g/L ou à une concentration de 5µg/cm² à 500 µg/cm².

10. Utilisation d'une lactonase selon la revendication 1,
dans laquelle ledit matériel contaminé par lesdites bactéries ou susceptible de l'être est choisi parmi :
- des dispositifs médicaux tels que des pansements, des cathéters, des endoscopes, des implants, des nébulisateurs
- du matériel médical
- des surfaces immergées telles que des coques de bateaux, des infrastructures portuaires ou pétrolières pouvant être la cible de biofouling ou de biocorrosion,
- des installations industrielles telles que des tours aéroréfrigérées, des systèmes de climatisation, des bioréacteurs, des tuyauteries, des nébulisateurs, des brumisateurs, des bassins.

11. Utilisation d'une lactonase selon la revendication 1, dans laquelle la prévention et/ou le traitement des infections de plantes est le feu bactérien.

12. Utilisation d'une lactonase selon l'une quelconque des revendications 1 à 11, dans une composition formulée avec au moins un excipient approprié pour son utilisation sous forme de solution, d'huile, de suspension, d'émulsion, de nanoparticules, de liposomes, de granulés ou de surface fonctionnalisée.

## Patentansprüche

1. Verwendung einer mutierten Lactonase, die zur Familie der Phosphotriesterase-ähnlichen Lactonasen gehört, zur Erhöhung der Empfindlichkeit von Bakterien gegenüber antimikrobiellen Mitteln im Vergleich zur alleinigen Verwendung antimikrobieller Mittel,
wobei mindestens die Aminosäure Tryptophan, die sich am Anfang von Loop 8 befindet, durch die Aminosäure Isoleucin ersetzt ist,
und die mutierte Lactonase die Sequenz SEQ ID NO: 1 aufweist, wobei mindestens die Aminosäure Tryptophan W an Position 263 durch die Aminosäure Isoleucin I ersetzt ist,
und wobei die Empfindlichkeit der Bakterien gegenüber antimikrobiellen Mitteln um mindestens den Faktor 2 erhöht ist,
wobei das antimikrobielle Mittel in einer Konzentration von 10 µM bis 100 mM verwendet wird,
die mutierte Lactonase in einer Konzentration von 0,1 mg/L bis 10 g/L oder in einer Konzentration von 1 µg/cm² bis 1 mg/cm² verwendet wird,
wobei das antimikrobielle Mittel ausgewählt ist aus der Gruppe, bestehend aus Antibiotika oder einem Gemisch von Antibiotika, Desinfektionsmitteln oder einem Gemisch von Desinfektionsmitteln, Bioziden oder einem Gemisch von Bioziden sowie aus Bakteriophagen, die gegebenenfalls natürlich in der Umwelt vorkommen oder nicht, oder einem Cocktail solcher Bakteriophagen,
und wobei die Verwendung ausgewählt ist aus
- der Vorbeugung und/oder Behandlung von Pflanzeninfektionen,
- einer Anwendung auf Material, das mit diesen Bakterien kontaminiert ist oder kontaminiert werden könnte, und
- einer Verwendung als Nahrungsergänzungsmittel für Menschen oder Tiere oder als Tierfuttermittel.

2. Verwendung einer Lactonase nach Anspruch 1, wobei das Antibiotikum ausgewählt ist aus der Gruppe, bestehend aus: Amikacin, Amoxicillin, Amoxicillin/Clavulanat, Ampicillin, Amprolium, Apramycin, Aspoxicillin, Aureomycin, Avilamycin, Azithromycin, Bacitracin, Bambermycin, Baquiloprim, Benzylpenicillin, Bicozamycin, Carbadox, Cefacetril, Cefalexin, Cefalonium, Cefalotin, Cefapyrin, Cefazolin, Cefdinir, Cefquinom, Ceftiofur, Ceftriaxon, Cefuroxim, Chloramphenicol, Chlortetracyclin, Ciprofloxacin, Clarithromycin, Clindamycin, Cloxacillin, Colistin, Dalbavancin, Danofloxacin, Decoquinat, Diclazuril, Dicloxacillin, Difloxacin, Doripenem, Doxycyclin, Enramycin, Enrofloxacin, Ertapenem, Erythromycin, Florfenicol, Flumequin, Fosfomycin, Framycetin, Fusidinsäure, Gentamicin, Gentamicinsulfat, Gramicidin, Halofuginon-Bromhydrat, Hetacillin, Imipenem, Imipenem/Cilastatin, Josamycin, Kanamycin, Kitasamycin, Laidlomycin, Lasalocid, Levofloxacin, Lincomycin, Lincomycin-Hydrochlorid, Maduramycin, Marbofloxacin, Mecillinam, Meropenem, Miloxacin, Minocyclin, Mirosamycin, Monensin, Moxifloxacin,
Nafcillin, Nalidixinsäure, Narasin, Neomycin, Neomycin/Oxytetracyclin, Neosporin, Nicarbazin, Norfloxacin, Novobiocin, Ofloxacin, Orbifloxacin, Oritavancin, Oxacillin, Oxolinsäure, Oxytetracyclin, Paromomycin, Penethamat-Hydroxid, Penicillin, Penicillin G-Kalium, Procain-Penicillin, Penicillin V-Kalium, Phenethicillin, Phenoxymethylpenicillin, Pirlimycin, Polymyxin, Polymyxin B, Polysporin (Bacitracin/Polymyxin), Pristinamycin, Rifampicin, Rifaximin, Roxarson, Salinomycin, Semduramicin, Spectinomycin, Spiramycin, Streptomycin, Sulfachlorpyridazin, Sulfadiazin, Sulfadimerazin, Sulfadimethoxazol, Sulfadimethoxin, Sulfadimethoxin und Ormetoprim 5:3, Sulfadimidin, Sulfadoxin, Sulfafurazol, Sulfaguanidin, Sulfamethazin, Sulfamethoxazol/Trimethoprim, Sulfamethoxin, Sulfamethoxypyridazin, Sulfamonomethoxin, Sulfanilamid, Sulfaquinoxalin, Sulfasalazin, Sulfisoxazol, Surfactin, Telavancin, Terdecamycin, Tetracyclin, Thiamphenicol, Tiamulin, Ticarcillin, Tilmicosin, Tobicillin, Tobramycin, Trimethoprim, Trimethoprim/Sulfonamid, Tulathromycin, Tylosin, Valnemulin, Vancomycin, Virginiamycin.

3. Verwendung einer Lactonase nach Anspruch 1, wobei das Desinfektionsmittel einen Alkohol, Chlor, einen Aldehyd, ein Oxidationsmittel, Iod, Ozon, eine phenolische Verbindung, eine quaternäre Ammoniumverbindung oder ein Gemisch aus zwei oder mehreren davon umfasst,
wobei das Desinfektionsmittel vorzugsweise Formaldehyd, Orthophthalaldehyd, Glutaraldehyd, Silberdihydrogencitrat, Polyaminopropylbiguanid, Natriumbicarbonat, Milchsäure, ein chlorhaltiges Bleichmittel, Methanol, Ethanol, n-Propanol, 1-Propanol, 2-Propanol, Isopropanol, ein Hypochlorit, Chlordioxid, Di-Chlorisocyanurat, Monochlorisocyanurat, Hydantoin, Natriumhypochlorit, Kalziumhypochlorit, Natrium-di-chlorisocyanurat, Natriumchlorit, 4-Methylbenzolsulfonamid, Natriumsalz, 2,4-Dichlorbenzylalkohol, Performinsäure, Paressigsäure, Kaliumpermanganat, Kaliumperoxymonosulfat, Phenol, Phenylphenol, Chloroxylenol, Hexachlorophen, Thymol, Amylmetakresol, Benzalkoniumchlorid, Cetyltrimethylammoniumbromid, Cetylpyridiniumchlorid, Benzethoniumchlorid, Borsäure, Brillantgrün, Chlorhexidin-Gluconat, Povidon-Iod, Mercurochrom, Manuka-Honig, Octenidin-Dihydrochlorid, Polyhexamethylenbiguanid, Perubalsam, Wasserstoffperoxid, organisches Peroxid, Peroxysäure, organisches Hydroperoxid, Peroxidsalz, Säureperoxide und Gemische aus zwei oder mehreren davon umfasst.

4. Verwendung einer Lactonase nach Anspruch 1, wobei das Biozid ausgewählt ist aus der Gruppe, bestehend aus:
Peroxiden mit biozider Wirkung wie Wasserstoffperoxid, ein- und mehrfunktionellen Alkoholen, Aldehyden, Säuren, Ozon, Naphtha-Verbindungen und Verbindungen, die ein Alkalimetall, ein Übergangsmetall, ein Metall der Gruppe III oder der Gruppe IV, ein Schwefel-, Stickstoff- oder Halogenatom enthalten, sowie Gemischen aus zwei oder mehreren davon,
wobei das Biozid vorzugsweise ausgewählt ist aus der Gruppe, bestehend aus: Formaldehyd, Glutaraldehyd, Peressigsäure, Alkalimetallhypochloriten, quaternären Ammoniumverbindungen, 2-Amino-2-methyl-1-propanol, Cetyltrimethylammoniumbromid, Cetylpyridiniumchlorid, 2,4,4-Trichlor-2-hydroxy-diphenylether, 1-(4-Chlorphenyl)-3-(3,4-dichlorphenyl)harnstoff, Zinkoxid, Zinkricinoleat, Pentachlorphenol, Kupfernaphthenat, Tributylzinnoxid, Dichlorophen, p-Nitrophenol, p-Chlor-m-xylenol, Beta-Naphthol, 2,3,5,6-Tetrachlor-4-(methylsulfonyl)pyridin, Salicylanilid, Bromessigsäure, quaternärem Alkylammoniumacetat, Natriumethylquecksilberthiosalicylat,
Natriumorthophenylphenat, n-Alkyl-(C₂-Cs)-dimethylbenzylammoniumchlorid, Organoboraten, 2,2-(1-Methyltrimethylendioxy)-bis-(4-methyl-1,3,2-dioxaborinan), 2,2-Oxybis(4,4,6-trimethyl)-1,3,2-dioxaborinan, Ethylenglykolmonomethylether, Parahydroxybenzoaten, organischen Borverbindungen, 8-Hydroxychinolin, Natrium-Pentachlorphenat, Alkyldimethylethylbenzyl-Ammoniumchlorid, Alkylammoniumsalzen, 1,3,5-Triethylhexahydro-1,3,5-triazin, Strontiumchromat, halogenierten Phenolen, 2-Brom-4-phenylphenol, Silbersalzen wie Silbernitrat, Silberchlorid, Silberoxid und elementarem Silber, organischen Peroxiden, Silbersulfadiazin, Natriumdichlor-S-triazinetrion, 4-Chlor-2-cyclohexylphenol, 2-Chlor-4-nitrophenol, Paraffinersatz, 3-Chlor-3-nitro-2-butanol, 2-Chlor-2-nitro-1-butanol-stearat, 2-Chlor-2-nitrobutylacetat, 4-Chlor-4-nitro-3-hexanol, 1-Chlor-1-nitro-1-propanol, 2-Chlor-2-nitro-1-propanol, Triethylzinnchlorid, 2,4,5-Trichlorphenol, 2,4,6-Trichlorphenol, 1,3-Dichlor-5,5-dimethylhydantoin, Tris(hydroxymethyl)nitromethan, Nitroparaffinen, 2-Nitro-2-ethyl-1,3-propandiol, 2-Ethyl-2-nitro-1,3-propandiol, 2-Methyl-2-nitro-1,3-propandiol, Hexahydro-1,3,5-tris(2-hydroxyethyl)-S-triazin, Hexahydro-1,3,5-tris(tetrahydro-2-furanyl)-methyl-S-triazin, Methylenbis(thiocyanat), 2,2-Dibrom-3-nitrilopropionamid, Beta-Brom-3-nitrostyrol, Fluorverbindungen, N-Ethyl-N-methyl-4-(trifluormethyl)-2-(3,4-dimethoxyphenyl)benzamid, Pentachlorphenol, Dichlorophen, Orthophenylphenol, Dibicyclo(3,1,1 oder 2,2,1)-heptyl-Polyaminen, Dibicyclo-(3,1,1 oder 2,2,1)-heptanyl-Polyaminen und Gemischen aus zwei oder mehreren davon.

5. Verwendung einer Lactonase nach Anspruch 1, wobei der Bakteriophage zur Familie der Myoviridae, Siphoviridae, Podoviridae, Corticoviridae, Cystoviridae, Inoviridae, Leviviridae, Microviridae, Plasmaviridae und Tectiviridae gehört oder einem Cocktail davon.

6. Verwendung einer Lactonase nach Anspruch 1, wobei die wirksame Dosis des antimikrobiellen Mittels im Vergleich zur wirksamen Dosis des antimikrobiellen Mittels allein um mindestens den Faktor 2 verringert ist.

7. Verwendung einer Lactonase nach Anspruch 1, wobei die wirksame Dosis des antimikrobiellen Mittels in der Zusammensetzung in einer Menge vorliegt, die mindestens zweimal geringer ist als die des mindestens einen antimikrobiellen Mittels allein.

8. Verwendung einer Lactonase nach Anspruch 1, wobei das antimikrobielle Mittel in einer Konzentration von 1 mM bis 100 mM verwendet wird.

9. Verwendung einer Lactonase nach Anspruch 1, wobei die mutierte Lactonase in einer Konzentration von 10 mg/l bis 2 g/l oder in einer Konzentration von 5 µg/cm² bis 500 µg/cm² verwendet wird.

10. Verwendung einer Lactonase nach Anspruch 1, wobei das mit den genannten Bakterien kontaminierte oder dafür anfällige Material ausgewählt ist aus
- medizinischen Produkten wie Verbänden, Kathetern, Endoskopen, Implantaten, Verneblern,
- medizinischem Material,
- unter Wasser liegenden Oberflächen wie Schiffsrümpfe, Hafen- oder Ölindustrieanlagen, die von Biofouling oder Biokorrosion betroffen sein können,
- industriellen Anlagen wie Kühltürme, Klimaanlagen, Bioreaktoren, Rohrleitungen, Vernebler, Sprühnebelanlagen und Becken.

11. Verwendung einer Lactonase nach Anspruch 1, wobei die Vorbeugung und/oder Behandlung von Pflanzeninfektionen den Feuerbrand betrifft.

12. Verwendung einer Lactonase nach einem der Ansprüche 1 bis 11 in einer Zusammensetzung, die mit mindestens einem für ihre Verwendung geeigneten Trägerstoff in Form einer Lösung, eines Öls, einer Suspension, einer Emulsion, von Nanopartikeln, Liposomen, Granulaten oder einer funktionalisierten Oberfläche formuliert ist.

## Claims

1. Use of a mutated lactonase belonging to the phosphotriesterase-like lactonase family to increase the susceptibility of bacteria to antimicrobial agents compared to the use of antimicrobial agents alone,
wherein at least the amino acid tryptophan at the beginning of loop 8 is substituted by the amino acid isoleucine,
and, said mutated lactonase having the sequence SEQ ID NO: 1 wherein at least the amino acid tryptophan W at position 263 is substituted by the amino acid isoleucine I, and, said susceptibility of bacteria to antimicrobial agents is increased by at least a factor of 2,
said antimicrobial agent is used at a concentration from10 µM to 100 mM,
said mutated lactonase is used at a concentration from 0,1 mg/L to 10 g/L or at a concentration from 1µg/cm² to 1 mg/cm²,
wherein the antimicrobial agent is selected from the group consisting of antibiotics or a mixture of antibiotics, disinfectant agents or a mixture of disinfecting agents, biocides or a mixture of biocides and bacteriophages that may or may not be naturally present in the environment, or a cocktail of such bacteriophages, and
wherein the use is chosen from
- prevention and/or treatment of plant infections,
- application to material contaminated or likely to be contaminated with said bacteria, and
- use as a food supplement for humans or animals or as an animal nutrition product.

2. The use of a lactonase according to claim 1, wherein said antibiotic is selected from the group consisting of: Amikacin, Amoxicillin, Amoxicillin/clavulanate, Ampicillin, Amprolium, Apramycin, Aspoxicillin, Aureomycin, Avilamycin, Azithromycin, Bacitracin, Bambermycin, Baquiloprim, Benzylpenicillin, Bicozamycin, Carbadox, Cefacetrile, Cefalexin, Cefalonium, Cefalonium, Cefalotin, Cefapyrine, Cefazoline, Cefdinir, Cefquinoma, Ceftiofur, Ceftriaxone, Cefuroxime, Chloramphenicol, Chlortetracycline, Ciprofloxacin, Clarithromycin, Clindamycin, Cloxacillin, Colistin, Dalbavancin, Danofloxacin, Decoquinate, Diclazuril, Dicloxacillin, Difloxacin, Doripenem, Doxycycline, Enramycin, Enrofloxacin, Ertapenem, Erythromycin, Florfenicol, Flumequine, Fosfomycin, Framycetin, Fusidic Acid, Gentamicin,
Gentamicin Sulfate, Gramicidin, Halofuginone Hydrobromide, Hetacillin, Imipenem, Imipenem/Cilastatin, Josamycin, Kanamycin, Kitasamycin, Laidlomycin, Lasalocid, Levofloxacin, Lincomycin, Lincomycin Hydrochloride, Maduramycin, Marbofloxacin, Mecillinam, Meropenem, Miloxacin, Minocycline, Mirosamycin, Monensin, Moxifloxacin, Nafcillin, Nalidixic Acid, Narasine, Neomycin, Neomycin/Oxytetracycline, Neosporine, Nicarbazine, Norfloxacin, Novobiocin, Ofloxacin, Orbifloxacin, Oritavancin, Oxacillin, Oxolinic Acid, Oxytetracycline, Paromomycin, Penethamate Hydroxide, Penicillin, Penicillin G Potassium, Penicillin Procaine, Penicillin V Potassium, Phenethicillin, Phenoxymethylpenicillin, Pirlimycin, Polymyxin, Polymyxin B, Polysporine (bacitracin/polymyxin), Pristinamycin, Rifampicin, Rifaximin, Roxarsone, Salinomycin, Semduramicin, Spectinomycin, Spiramycin, Streptomycin, Sulfachlorpyridazine, Sulfadiazine, Sulfadimerazine, Sulfadimethoxazole, Sulfadimethoxine, Sulfadimethoxine and Ormetoprim 5:3, Sulfadimidine, Sulfadoxine, Sulfafurazole, Sulfaguanidine, Sulfamethazine, Sulfamethoxazole/trimethoprim, Sulfamethoxin, Sulfamethoxypyridazine, Sulfamonomethoxin, Sulfanitramide, Sulfaquinoxaline, Sulfasalazine, Sulfaxazole, Surfactin, Telavancin, Terdecamycin, Tetracycline, Thiamphenicol, Tiamulin, Ticarcillin, Tilmicosin, Tobicillin, Tobramycin, Trimethoprim, Trimethoprim/Sulfonamide, Tulathromycin, Tylosin, Valnemuline, Vancomycin, Virginiamycin.

3. The use of a lactonase according to claim 1, wherein said disinfectant agent comprises an alcohol, chlorine, aldehyde, oxidizing agent, iodine, ozone, phenolic compound, quaternary ammonium compound or a mixture of two or more of these, preferably, said disinfectant agent includes formaldehyde, orthophthalaldehyde, glutaraldehyde, silver dihydrogen citrate, polyaminopropyl biguanide, sodium bicarbonate, lactic acid, chlorinated bleach, methanol, ethanol, n-propanol, 1-propanol, 2-propanol, isopropanol, hypochlorite, chlorine dioxide, di chloro isocyanurate, monochloro isocyanurate, hydantoin, sodium hypochlorite, calcium hypochlorite, sodium dichloro isocyanurate, sodium chlorite, 4-methylbenzenesulfonamide, sodium salt, 2,4-benzyl dichlorobenzyl alcohol, performic acid, paracetic acid, potassium permanganate, potassium peroxymonosulfate, phenol, phenylphenol, chloroxylenol, hexachlorophene, thymol, amylmetacresol, benzalkonuim chloride, cetyltrimethylammonium bromide, cetylpyridinium chloride, benzethonium chloride, boric acid, bright green, chlorhexidine gluconate, providone iodine, mercurochrome, manuka honey, octenidine dihydrochloride, polyhexamethylene biguanide, balsam of Peru, hydrogen peroxide, organic peroxide, peroxyacid, organic hydroperoxide, peroxide salt, acid peroxides, and mixtures of two or more of the these.

4. The use of a lactonase according to claim 1, wherein the biocide is selected from the group consisting of: active biocidal peroxides such as hydrogen peroxide, mono and polyfunctional alcohols, aldehydes, acids, ozone, naphtha compounds, and compounds containing an alkali metal, a transition metal, a group III or group IV metal, a sulphur, nitrogen or halogen atom and mixtures of two or more of these,
preferably, said biocide is selected from the group consisting of: formaldehyde, glutaraldehyde, peracetic acid, alkali metal hypochlorites, quaternary ammonium compounds, 2-amino-2-methyl-1-propanol, cetyltrimethylammonium bromide, cetylpyridinium chloride, 2,4,4-trichloro-2-hydroxy diphenyl ether, 1-(4-chlorophenyl)-3-(3,4-dichlorophenyl) urea, zinc oxide, zinc ricinoleate, pentachlorophenol, copper naphthenate, tributyltin oxide, dichlorophene, p-nitrophenol, p-chloro-m-xylenol, beta-naphthol, 2,3,5,6-tetrachloro-4-(methylsulfonyl)pyridine, salicylanilide, bromoacetic acid, alkyl quaternary ammonium acetate, ethyl thiosalicylate sodium mercury, sodium orthophenylphenate, n-alkyl (C2 to Cs) dimethyl benzyl ammonium chloride, organoborates, 2,2-(1-methyltrimethylene dioxy)-bis-(4-methyl-1,3,2-dioxaborinan), 2,2-oxybis(4,4,6-trimethyl)-1,3,2-dioxaborinan, ethylene glycol monomethyl ether, parahydroxybenzoates, organic compounds of boron, 8-hydroxyquinoline, sodium pentachlorophenate, alkyl dimethyl ethyl benzyl ammonium chloride, alkylammonium salts, 1,3,5-triethylhexahydro-1,3,5-triazine, strontium chromate, halogenated phenols, 2-bromo-4-phenylphenol, silver salts such as silver nitrate, silver chloride, silver oxide and elemental silver, organic peroxides, silver sulfadiazine, sodium dichloro-S-triazinetrione, 4-chloro-2-cyclohexylphenol, 2-chloro-4-nitrophenol, paraffin substitute, 3-chloro-3-nitro-2-butanol, 2-chloro-2-nitro-1-butanol stearate, 2-chloro-2-nitrobutyl acetate, 4-chloro-4-nitro-3-hexanol, 1-chloro-1-nitro-1-propanol, 2-chloro-2-nitro-1-propanol, triethyltin chloride, 2,4,5-trichlorophenol, 2,4,6-trichlorophenol, 1,3-dichloro-5,5-dimethylhydantoin, tris(hydoxymethyl)nitromethane, nitroparaffins, 2-nitro-2-ethyl-1,3-propanediol, 2-ethyl-2-nitro-1,3-propanediol, 2- methyl-2-nitro-1,3-propanediol, hexahydro-1,3,5-tris(2-hydroxyethyl)-S-triazine, hexahydro-1,3,5-tris(tetrahydro-2-furanyl)-methyl-S-triazine, methylene bis(thiocyanate), 2,2-dibromo-3-nitrilopropionamide, beta-bromo-3-nitrostyrene, fluorinated compounds, N-ethyl-N-methyl-4-(trifluoromethyl)-2-(3,4-dimethoxyphenyl)benzamide, pentachlorophenol, dichlorophene, orthophenylphenol, di-bicyclo (3,1,1 or 2,2,1)-heptyl polyamines, di-bicyclo-(3,1,1 or 2,2,1)-heptanyl polyamines and mixtures of two or more of these.

5. The use of a lactonase according to claim 1, wherein the bacteriophage belongs to the family Myoviridae, Siphoviridae, Podoviridae, Corticoviridae, Cystoviridae, Inoviridae, Leviviridae, Microviridae, Plasmaviridae and Tectiviridae, or a cocktail thereof.

6. The use of a lactonase according to claim 1, wherein the effective dose of antimicrobial agent is decreased by at least a factor of 2 compared to the effective dose of antimicrobial agent alone.

7. The use of a lactonase according to claim 1, wherein the effective dose of an antimicrobial agent in said composition being present in an amount at least 2 times lower than that of said at least one antimicrobial agent alone.

8. The use of a lactonase according to claim 1, wherein said antimicrobial agent is used at a concentration from 1 mM to 100 mM.

9. The use of a lactonase according to claim 1, wherein said mutated lactonase is used at a concentration from 10 mg/L to 2 g/L or at a concentration from 5µg/cm² to 500 µg/cm².

10. Use of a lactonase according to claim 1,
wherein the said material contaminated with said bacteria or likely to be contaminated is selected from:
- medical devices such as dressings, catheters, endoscopes, implants, nebulizers
- medical equipment
- submerged surfaces such as boat hulls, port or oil infrastructure that may be the target of biofouling or biocorrosion,
- industrial facilities such as air-cooled towers, air conditioning systems, bioreactors, piping, foggers, misters, ponds.

11. The use of a lactonase according to claim 1, wherein the prevention and/or treatment of plant infections is fire blight.

12. The use of a lactonase according to any one of claims 1 to 11, in a composition formulated with at least one excipient suitable for use under the form of a solution, of an oil, of a suspension, of an emulsion, of nanoparticles, of liposomes, of granules, or of functionalized surface.
